(19) 

Europäisches
Patentamt
European
Patent Office
Office européen
des brevets

(11) **EP 2 896 686 A1**

(12) **EUROPEAN PATENT APPLICATION**

(43) Date of publication:
**22.07.2015 Bulletin 2015/30**

(21) Application number: **15153776.8**

(22) Date of filing: **02.07.2010**

(51) Int Cl.:
***C12N 1/14*** *(2006.01)*     ***C12N 9/42*** *(2006.01)*
***C12R 1/645*** *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **03.07.2009  EP 09164516
16.04.2010  EP 10160187**

(83) **Declaration under Rule 32(1) EPC (expert
solution)**

(62) Document number(s) of the earlier application(s) in
accordance with Art. 76 EPC:
**10734942.5 / 2 449 087**

(27) Previously filed application:
**02.07.2010 PCT/EP2010/059483**

(71) Applicant: **DSM IP Assets B.V.
6411 TE Heerlen (NL)**

(72) Inventors:
• **Perkins, John B.**
 **6100 AA Echt (NL)**
• **Kumar, Manoj**
 **6100 AA Echt (NL)**
• **Zijl, Van, Margrieta Frederique Kim**
 **6100 AA Echt (NL)**
• **Vollebregt, Adrianus Wilhelmus Hermanus**
 **6100 AA Echt (NL)**
• **Sarantinopoulos, Panagiotis**
 **6100 AA Echt (NL)**

(74) Representative: **Biermann, Jan et al
DSM Intellectual Property
P.O. Box 4
6100 AA Echt (NL)**

Remarks:
This application was filed on 04-02-2015 as a
divisional application to the application mentioned
under INID code 62.

(54) **Talaromyces strains and enzyme compositions**

(57)     The present invention relates to *Talaromyces* strains. The invention further relates to enzyme compositions, which may be produced by the *Talaromyces* strains. Further the invention relates to methods for producing useful products from lignocellulosic material using the enzyme compositions.

EP 2 896 686 A1

**Description**

**Field of the invention**

The invention relates to *Talaromyces* strains and mutants with improved cellulase production. The invention further relates to enzyme compositions, which may be produced by the *Talaromyces* strains. Further the invention relates to methods for producing useful products from (ligno)cellulosic material using the enzyme compositions.

**Background of the invention**

Carbohydrates constitute the most abundant organic compounds on earth. However, much of this carbohydrate is sequestered in complex polymers including starch (the principle storage carbohydrate in seeds and grain), and a collection of carbohydrates and lignin known as lignocellulose. The main carbohydrate components of lignocellulose are cellulose, hemicellulose, and pectins. These complex polymers are often referred to collectively as lignocellulose.

Bioconversion of renewable lignocellulosic biomass to a fermentable sugar that is subsequently fermented to produce alcohol (e.g., ethanol) as an alternative to liquid fuels has attracted an intensive attention of researchers since 1970s, when the oil crisis broke out because of decreasing the output of petroleum by OPEC. Ethanol has been widely used as a 10% blend to gasoline in the USA or as a neat fuel for vehicles in Brazil in the last two decades. More recently, the use of E85, an 85% ethanol blend has been implemented especially for clean city applications. The importance of fuel bioethanol will increase in parallel with increases in prices for oil and the gradual depletion of its sources. Additionally, fermentable sugars are being used to produce plastics, polymers and other biobased products and this industry is expected to grow substantially therefore increasing the demand for abundant low cost fermentable sugars which can be used as a feed stock in lieu of petroleum based feedstocks.

The sequestration of such large amounts of carbohydrates in plant biomass provides a plentiful source of potential energy in the form of sugars, both five carbon and six carbon sugars that could be utilized for numerous industrial and agricultural processes. However, the enormous energy potential of these carbohydrates is currently under-utilized because the sugars are locked in complex polymers, and hence are not readily accessible for fermentation. Methods that generate sugars from plant biomass would provide plentiful, economically-competitive feedstocks for fermentation into chemicals, plastics, and fuels.

Regardless of the type of cellulosic feedstock, the cost and hydrolytic efficiency of enzymes are major factors that restrict the commercialization of the biomass bioconversion processes. The production costs of microbially produced enzymes are tightly connected with a productivity of the enzyme-producing strain and the final activity yield in the fermentation broth.

Cellulases are enzymes that hydrolyze cellulose (beta-1,4-glucan or beta D-glucosidic linkages) resulting in the formation of glucose, cellobiose, cellooligosaccharides, and the like.

Cellulases have been traditionally divided into three major classes: endoglucanases (EC 3.2.1.4) ("EG"), exoglucanases or cellobiohydrolases (EC 3.2.1.91) ("CBH") and beta-glucosidases ([beta]-D-glucoside glucohydrolase; EC 3.2.1.21) ("BG"). See e.g. Knowles et al., TIBTECH 5, 255-261, 1987; Shulein, Methods Enzymol., 160, 25, pp. 234-243, 1988. Endoglucanases act mainly on the amorphous parts of the cellulose fibre, whereas cellobiohydrolases are also able to degrade crystalline cellulose (Nevalainen and Penttila, Mycota, 303-319, 1995). Thus, the presence of a cellobiohydrolase in a cellulase system is required for efficient solubilization of crystalline cellulose (Suurnakki, et al. Cellulose 7:189-209, 2000). Beta-glucosidase acts to liberate D-glucose units from cellobiose, cello-oligosaccharides, and other glucosides (Freer, J. Biol. Chem. vol. 268, no. 13, pp. 9337-9342, 1993).

Cellulases are known to be produced by a large number of bacteria, yeast and fungi. Certain fungi produce a complete cellulase system capable of degrading crystalline forms of cellulose, such that the cellulases are readily produced in large quantities via fermentation. Filamentous fungi play a special role since many yeast, such as Saccharomyces cerevisiae, lack the ability to hydrolyze cellulose. See, e.g., Aubert et al., 1988; Wood et al., Methods in Enzymology, vol. 160, no. 9, pp. 87-116, 1988, and Coughlan, et al., "Comparative Biochemistry of Fungal and Bacterial Cellulolytic Enzyme Systems" Biochemistry and Genetics of Cellulose Degradation, pp. 11-30 1988.

Fungal cellulase classifications of CBH, EG and BG can be further expanded to include multiple components within each classification. For example, multiple CBHs, EGs and BGs have been isolated from a variety of fungal sources including Trichoderma reesei which contains known genes for 2 CBHs, i.e., CBH I and CBH II, at least 8 EGs, i.e., EG I, EG II, EG III, EGIV, EGV, EGVI, EGVII and EGVIII, and at least 5 BGs, i.e., BG1, BG2, BG3, BG4 and BG5.

In spite of the continued research of the last few decades to understand enzymatic lignocellulosic biomass degradation and cellulase production, it remains desirable to discover or to engineer new highly active cellulases and hemicellulases. It would also be highly desirable to construct highly efficient enzyme compositions capable of performing rapid and efficient biodegradation of lignocellulosic materials.

In addition, currently available enzymes having cellulase activity, typically derived from Trichoderma, function at mesophilic temperatures, such as from 45°C to 50°C and at pH 5.0. This, however, may lead to bacterial infection reducing product yield, so it is desirable to carry out saccharification at a temperature of 65°C or higher. In addition, the use of mesophilic temperatures increases the viscosity of the biomass being used such that the dry matter content used is limited. Also, when acid pretreated biomass is used as a substrate, the pH must be raised so that the enzyme can saccharify the sugars in the biomass. In the context of a commercially viable fuel ethanol industry, this implies a requirement for, for example, sodium hydroxide or calcium sulphate and the production of huge quantities of the corresponding salts, for example gypsum in the case of sodium hydroxide. Accordingly, it is desirable to carry out saccharification using an enzyme which can operate at a pH of pH 4.0 or lower.

## Summary of the invention

The invention provides *Talaromyces* mutant strains that have improved cellulolytic activity of at least 6 $FPU_{2\%}$/ml. The mutant strains according to the invention show good growth and good sporulation.

The invention further relates to enzyme compositions comprising cellobiohydrolase (CBH) and endoglucanase (EG), wherein the composition comprises 40% or more of CBH and 20% or more EG. Preferably, the enzyme compositions have a protease activity of at most 60 APU/ml.

The enzyme compositions can extremely effectively hydrolyze lignocellulolytic material, for example corn stover or wheat straw, into monomeric sugars which can then be converted into a useful product, such as ethanol.

The enzyme compositions can be used to carry out highly effective hydrolysis of a lignocellulosic substrate. This is highly significant in the context of commercially viable fuel ethanol production from lignocellulosic biomass since lower amounts of enzyme will be required (as compared with currently available products).

Moreover, this hydrolysis may be carried out at a high temperature which (i) reduces the risk of bacterial infection and (ii) results in a less viscous biomass pulp. The effect of the latter is significant since it enables the better blending of enzymes, resulting in a higher operational dry matter in the plant and allows a consequent higher ethanol concentration to be achieved. Thus, less energy need be used improving sustainability and a smaller fermentation process will be required requiring lower investment.

Also, this hydrolysis may be carried out at low pH. This is desirable since biomass is often pretreated with acid. Biomass treated in this way does not have to be pH adjusted if the enzymes subsequently used for saccharification are capable of acting at low pH. This implies a lower requirement of, for example, sodium hydroxide or calcium sulphate and a process in which there is no waste salt. This is significant in a process in which, for example, fuel ethanol is to be produced since huge quantities of material are consumed in such processes. This allows a process to be carried out in which no pH adjustment is required, i.e. there is no requirement for the addition of acids or bases. The process may thus be carried out as a zero waste process and/or as a process in which no inorganic chemical input is required.

In addition, it has been shown that the enzyme composition can effectively hydrolyze biomass when high dry matter contents are used. It is highly desirable that enzymes used in the production of, for example, fuel ethanol are able to operate on substrates having high viscosity (i.e. high dry weight composition) since this allows higher amounts of the final product, for example, fuel ethanol, to be achieved.

Further the invention relates to methods for producing useful products from lignocellulosic material using the enzyme compositions.

## Brief description of the drawings

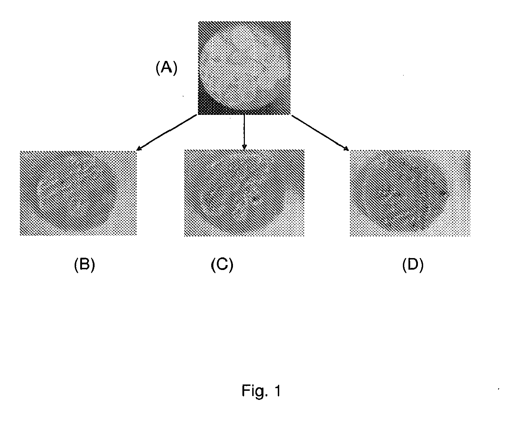

Fig. 1 shows different colony phenotypes of *Talaromyces emersonii* strain TEC-1A. A) TEC-1A; B) TEC-101, distinct white aerial mycelia (80% of population); C, TEC-102, reduced aerial mycelia (20% of population); TEC-104, no distinct aerial mycelia, brown color (1% of population).

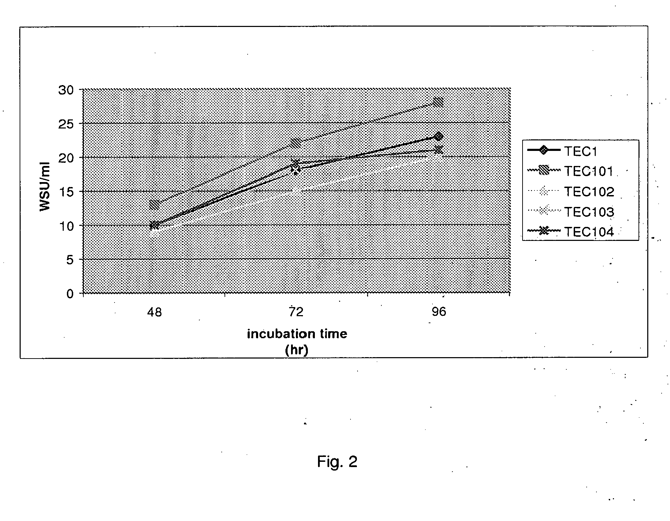

Fig. 2 shows the results of a course experiment in shake flasks with different colony morphology variants of TEC-1A strain grown using Talaromyces medium 2.

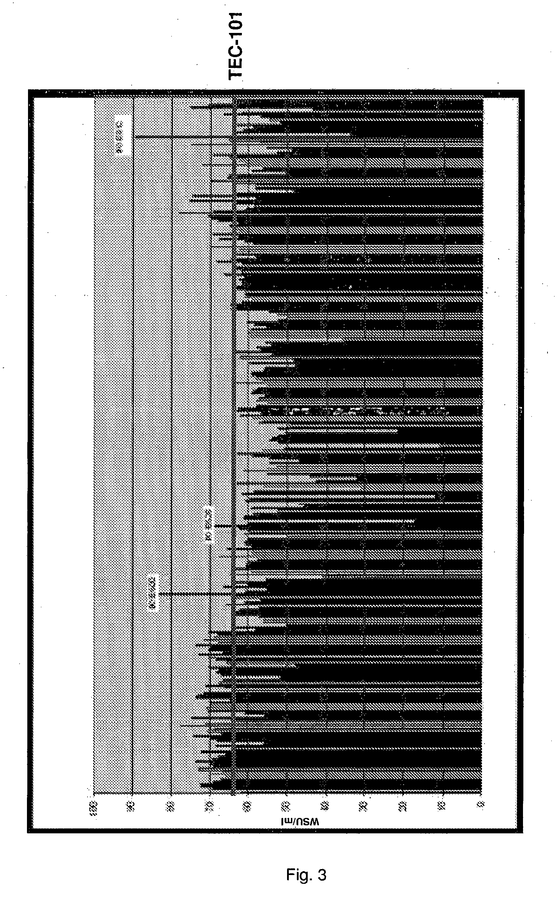

Fig. 3 shows the performance of NTG (N-methyl-N'-nitro-N-nitrosoguanidine)-induced cellulase production mutants of TEC-101 mutants in secondary shake flask tests (Talaromyces medium 2). Wheat straw assay was performed at pH 4.5, 20 hours at 65°C with undiluted supernatant. Interesting strains are accommodated with strain ID. Cellulase levels are expressed in wheat straw units per milliliter (WSU/ml). The cellulase production level from the parental control strain TEC-101 is indicated by the red line.

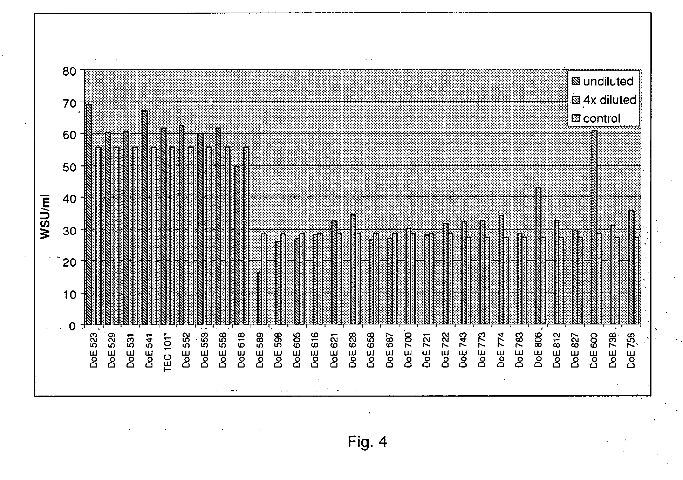

Fig. 4 shows a summary of tertiary screening results in shake flask. Fermentations in *Talaromyces* medium 2, 96 hours at 46°C using spores from agar slants. TEC-101* is a spontaneous mutant that produce higher than normal cellulase levels. Wheat straw assays were performed at pH 4.5 for 20 hours at 65°C. Blue bars are undiluted samples; pink bars are four times diluted samples. Orange bars are the cellulase production level from the TEC-101 parental control and is an average of two or three shake flask experiments.

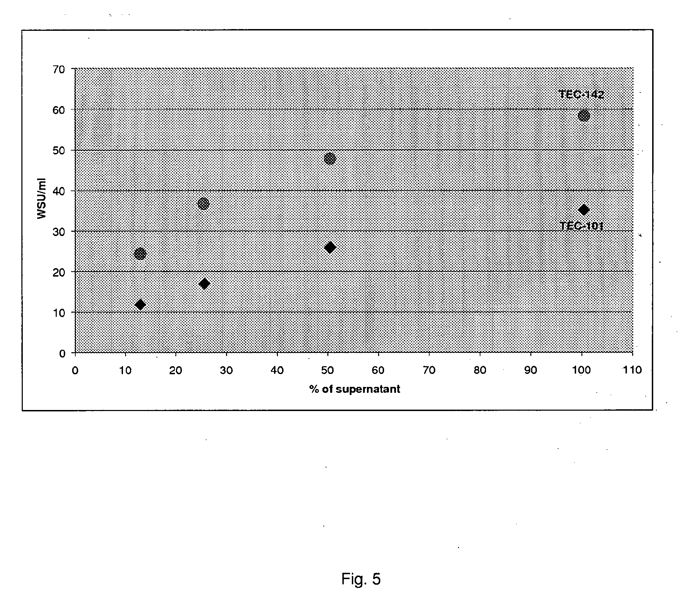

Fig. 5 shows the dose response of conversion of wheat straw. Strains tested in shake flask: TEC-142 (✧) and TEC-101 (♦). Cellulase activity is expressed in wheat straw units per milliliter (WSU/ml).

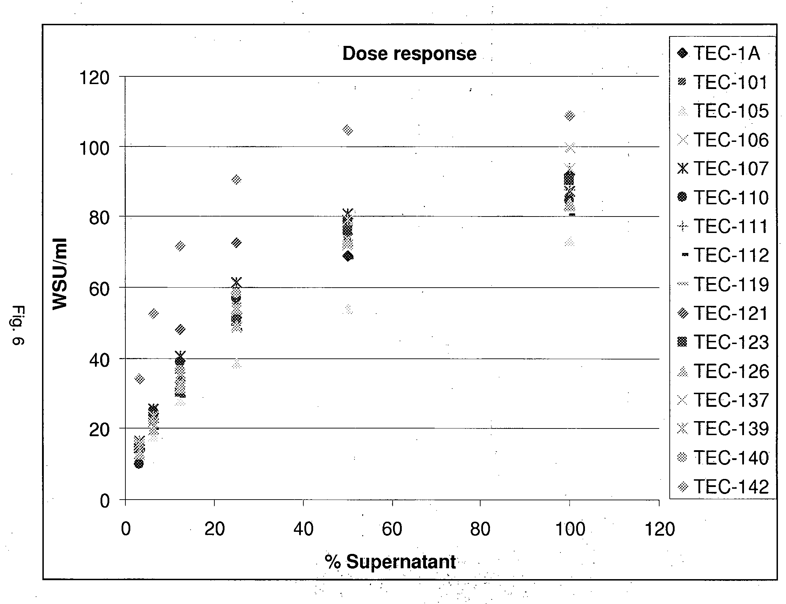

Fig. 6 shows the dose-response curve of diluted supernatants of shake flask cultures of mutants grown in Talaromyces medium 2 for 4 days at 46°C and assayed for cellulase levels in the wheat straw assay and expressed in wheat straw units per milliliter (WSU/ml).

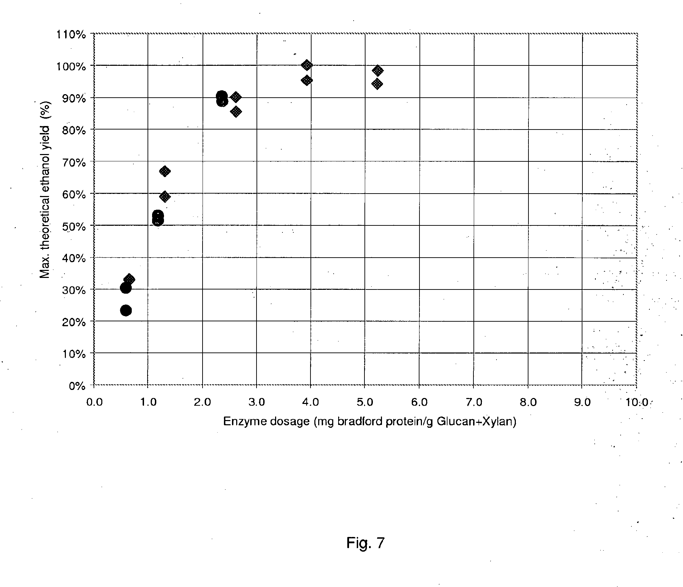

Fig. 7 shows comparison of ethanol yields of Filtrase® NL (♦) and supernatant of TEC-142 (●) in dose-response Simultaneous Saccharification Fermentation (SSF) experiments using pre-treated, wash wheat straw. TEC-142 was grown for 96 hr in 10-liter batch fermentations. Ethanol production is expressed as a percentage of the maximum theoretical yield of ethanol produced from 48.5% glucan fibre content (NREL) of the pre-treated wheat straw, which is labelled on the y-axis as "Max. theoretical ethanol yield (%)".

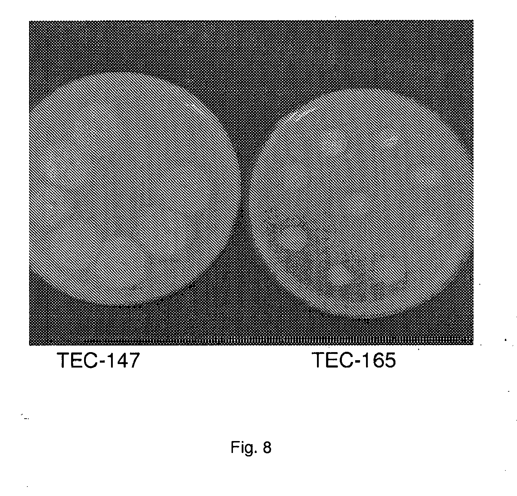

Fig. 8 shows a comparison of the growth of glucose derepressed enzyme mutant TEC-165 relative to its glucose repressed parent TEC-147. Spore solution ($10^7$/ml) where spotted onto TEC medium and grown at 40°C for 3-4 days. The size of the colony indicates the extent of growth.

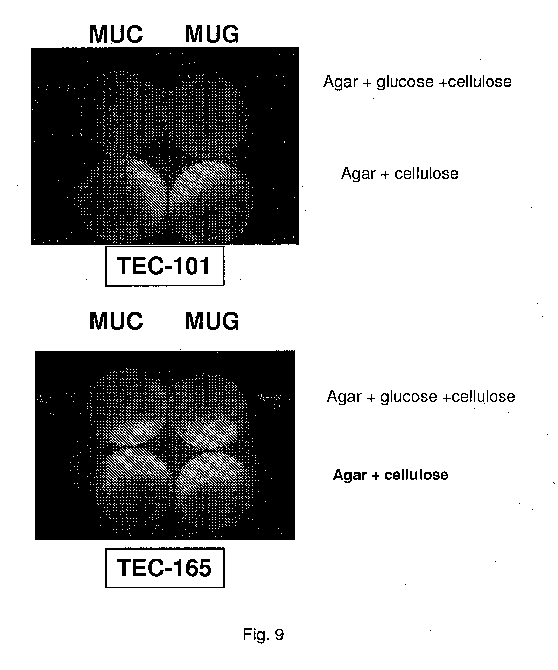

Fig. 9 shows a comparison of CBH I+II and β-glucosidase activities of TEC-165 vs TEC-101 using fluorescent substrates 4-methylumbelliferyl-β-D-cellobioside (MUC) or 4-methylumbelliferyl-β-D-glucose (MUG), respectively. Spore solution ($10^7$/ml) of each strain was streaked for single colonies onto duplicate TEC medium agar plates containing 0.1mM MUC or MUG with or without 2% glucose. Plates were incubated at 40°C for 1-2 days. Plates were photograph under UV light (366 nm). The light areas of the agar plate are the fluorescent (blue) signal caused by hydrolysis of the substrate by cellulases produced by the fungal growth; the size and intensity of the fluorescent signal indicates the extent of enzyme activity.

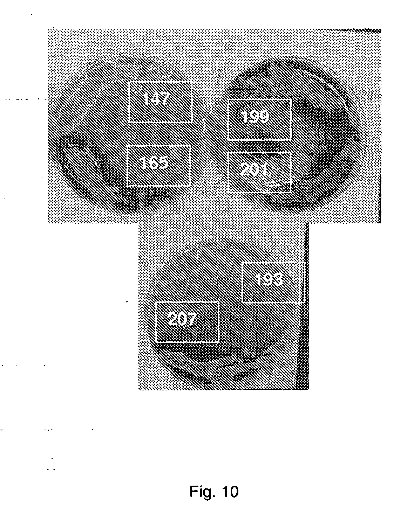

Figure 10 shows the comparison of xylanase activity of glucose derepressed mutants TEC-165, TEC-199, TEC-201, and TEC-207 to non-glucose derepressed parents TEC-147 and TEC-193 using the color substrates AZCL. Spore solution ($10^7$/ml) of each strain was streaked for single colonies onto TEC medium agar plates containing 0.1% AZCL and 2% glucose. Plates were incubated at 40°C for 3-4 days. The size and intensity of the dark areas (blue halo) surrounding the fungal growth indicates the extent of enzyme activity.

## Detailed description of the invention

Throughout the present specification and the accompanying claims, the words "comprise" and "include" and variations such as "comprises", "comprising", "includes" and "including" are to be interpreted inclusively. That is, these words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

The *Talaromyces* strains according to the invention have a cellulolytic activity of at least 6 $FPU_{2\%}$/ml. Preferably they have a cellulolytic activity of at least 7 $FPU_{2\%}$/ml, at least 8 or at least 9, more preferably at least 10, at least 11, at least 12, at least 13, at least 14, at least 15, art least 16, at least 17, at least 18, at least 19, at least 20 $FPU_{2\%}$/ml. Preferred ranges for cellulolytic activity are 6-300 $FPU_{2\%}$/ml, 6-150 $FPU_{2\%}$/mlor 10-75 $FPU_{2\%}$/ml. for EG activity 5000-200000 WBCU/ml, 5000-100000 WBCU/ml and 8000-50000 WBCU/ml. Cellulolytic activity is herein measured based on a modified method by Adney and Baker as is described in the examples and expressed in units abbreviated as $FPU_{2\%}$/ml. High cellulolytic activity is desirable as it will reduce the final cost of the enzyme composition that may be produced with the strain.

Preferably, the *Talaromyces* strains have an endoglucanase activity of at least 5000 WBCU/ml, more preferably at least 6000 WBCU/ml, more preferably at least 7000 WBCU/ml, more preferably at least 8000 WBCU/ml, more preferably at least 9000 WBCU/ml, even more preferably at least 10000 WBCU/ml, most preferably 10200 WBCU/ml or more. Preferred ranges for endoglucanase activity are 5000-200000 WBCU/ml, 5000-100000 WBCU/ml or 8000-50000 WBCU/ml. Endoglucanase activity of the strains is herein measured based on a method as is described in the examples and expressed in White Biotech Cellulase Units/ml abbreviated as WBCU/ml. High endoglucanase activity is desirable since endoglucanase activity will generate more enzymatic sites for the exo-glucanases (CBH I and II) present in the enzyme composition, so that a substrate may be degraded more quickly.

Preferably, the *Talaromyces* strains have low protease activity, in particular a low acid protease activity of at most 60 APU/ml, at most 50 APU/ml, at most 40 APU/ml, most preferably at most 35 APU/ml, at most 30 APU/ml, at most 20 APU/ml at most 10 APU/ml.. Acid protease activity is herein measured based on a method as is described in the examples and expressed in Acid Protease Units/ml abbreviated as APU/ml. Low protease activity is desirable will help generate a more (long term) stable enzyme composition.

Preferably, the *Talaromyces* strain has sporulation and produces a spore titer of at least $10^6$ / ml. Good sporulation facilitates further improvement of the strain by classical or genetic engineering methods.

The invention further relates to Mutant *Talaromyces* strains produced by mutation of a wild-type *Talaromyces* strain, wherein the protein levels of one or more of the proteins CBH I, CBH II, EG/CEA, EG/CEB, Xylanase, Acetyl Xylan Esterase, Swollenin-like protein, EG/ GH61 and/or carbohydrate degrading protein having a molecular weight of about 22993 Dalton is increased at least 1.5-fold relative to the protein level in the wild-type *Talaromyces* strain, preferably at least 2-fold,

In an embodiment thereof the CBH I has a molecular weight of about 48690 Daltons and/or the CBH II has a molecular weight of about 46674 Daltons and/or the EG/CEA has a molecular weight of about 36825 Daltons and/or the EG/CEB has a molecular weight of about 51462 Daltons and/or the acetyl xylan esterase has a molecular weight of about 48690 Daltons and/or the swollening-like enzyme has a molecular weight of about 67911 Daltons and/or the EG/ GH61, has a molecular weight of about 27007 Daltons.

Molecular weights are herein calculated on AA acid-sequence base and expressed in Daltons.

Protein levels are herein determined as described by APEX as described in example 14, with the exception of those situations wherein it is indicated that another method is used, e.g. SDS-PAGE or protease assay. In such situations the method will be executed as described in the examples.

In an embodiment, the *Talaromyces* strains according to the invention have a protein level of the proteins CBH I, CBH II, EG/CEA, EG/CEB, Xylanase, Acetyl Xylan Esterase, Swollenin-like protein, EG/GH61 and that of carbohydrate degrading protein having a molecular weight of about 22993 Dalton increased at least 1.5-fold, preferably increased at least 2-fold.

In an embodiment the mutant *Talaromyces* strains according to the invention have a protein level of one or more of proteins EG/ GH61, swollenin-like protein, and/or carbohydrate degrading protein (Temer1170) that is increased relative to the wild-type *Talaromyces* strain, preferably that level is increased at least 2-fold.

In an embodiment the mutant *Talaromyces* strains according to the invention have compared to the wild-type *Talaromyces* strain a protein level of one or more the proteins endoglucanase EG, xylosidase, chitanase and or protease, is decreased relative to the production in the wild-type *Talaromyces* strain, preferably production that is decreased at least 2-fold. In an embodiment the mutant *Talaromyces* strains according to the invention has an endo-glucanase EG that has a molecular weight of about 24136 Daltons and/or the xylosidase has a molecular weight of about 95022 Daltons and/or the chitanase has a molecular weight of about 45111 Daltons and/or the protease has a molecular weight of about 28800 Daltons.

In an embodiment the mutant *Talaromyces* strains according to the invention have a ratio of EG/GH61 protein level to the sum of the protein levels of a) EG, b) EG/CEA and c) EG/CEB together of at least 0.8, preferably at least 1.0.

In an embodiment the mutant *Talaromyces* strains according to the invention have production of the proteins CBH I, CBH II, EG/CEA, EG/CEB, Xylanase, Acetyl Xylan Esterase, Swollenin-like protein, and that of carbohydrate degrading protein Temer01170 that is not repressed by glucose.

In one embodiment, the *Talaromyces emersonii* strain is TEC-142 (also designated as FBG 142) deposited at CENTRAAL BUREAU VOOR SCHIMMELCULTURES, Uppsalalaan 8, P.O. Box 85167, NL-3508 AD Utrecht, The Netherlands on 1st July 2009 having the Accession Number CBS 124902, or an functionally equivalent mutant or descendent thereof.

In another embodiment., the strain is the *Talaromyces emersonii* strain TEC-192 (also designated as FBG 192) deposited at CENTRAAL BUREAU VOOR SCHIMMELCULTURES, Uppsalalaan 8, P.O. Box 85167, NL-3508 AD Utrecht, The Netherlands on 25th June 2010 having the Accession Number CBS 127389, or a functionally equivalent mutant or descendent thereof.

The invention further relates to enzyme compositions comprising cellobiohydrolase (CBH) and endoglucanase (EG), wherein the composition comprises 40% (Relative to the total protein based on an SDS-page image) or more of CBH and 20% (Relative to the total protein based on an SDS-page image) or more EG. Preferably the enzyme compositions have a protease activity is at most 60 APU/ml, at most 50 APU/ml, at most 40 APU/ml, or at most 35 APU/ml.

The invention further relates to a method for the conversion of lignocellulosic material into useful product comprising the following steps: a) pretreatment of one or more lignocellulosic material to produce pretreated lignocellulosic material; b) enzymatic treatment of the pretreated lignocellulosic material to produce one or more sugar; c) converting the sugar into one or more useful product and d) separating the one or more useful product, wherein in step a), b) or c) an enzyme composition is used or added.

Preferably in the method, the lignocellulosic material is orchard primings, chaparral, mill waste, urban wood waste, municipal waste, logging waste, forest thinnings, short- rotation woody crops, industrial waste, wheat straw, oat straw, rice straw, barley straw, rye straw, flax straw, soy hulls, rice hulls, rice straw, corn gluten feed, sugar cane, corn stover, corn stalks, corn cobs, corn husks, miscanthus, sweet sorghum, canola stems, soybean stems, prairie grass, gamagrass, foxtail; sugar beet pulp, citrus fruit pulp, seed hulls, cellulosic animal wastes, lawn clippings, cotton, seaweed, softwood, poplar, pine, shrubs, grasses, wheat, sugar cane bagasse, corn, corn hobs, corn kernel, fiber from kernels, products and by-products from wet or dry milling of grains, municipal solid waste, waste paper, yard waste, herbaceous material, agricultural residues, forestry residues, waste paper, pulp, paper mill residues, branches, bushes, canes, an energy crop, forest, a fruit, a flower, a grain, a grass, a herbaceous crop, a leaf, bark, a needle, a log, a root, a sapling, a shrub, switch grass, a tree, a vegetable, fruit peel, a vine, sugar beet pulp, wheat midlings, oat hulls, hard or soft wood, organic waste material generated from an agricultural process, forestry wood waste, or a combination of any two or more thereof. As described above step a) is pretreatment of one or more lignocellulosic material to produce pretreated lignocellulosic material. In the pretreatment step, the feedstock may be pre-treated with heat, mechanical and/or chemical modification or any combination of such methods in order to enhance the accessibility of the substrate to enzymatic hydrolysis and/or hydrolyse the hemicellulose and/or solubilize the hemicellulose and/or cellulose and/or lignin, in any way known in the art. The pretreatment may comprise exposing the lignocellulosic material to (hot) water, steam (steam explosion), an acid, a base, a solvent, heat, a peroxide, ozone, mechanical shredding, grinding, milling or rapid depressurization, or a combination of any two or more thereof. A chemical pretreatment is often combined with heat-pretreatment, e.g. between 150-220 °C for 1 to 30 minutes.

Preferably, in the method according to the invention, the useful product is one or more of ethanol, butanol, lactic acid, a plastic, an organic acid, a solvent, an animal feed supplement, a pharmaceutical, a vitamin, an amino acid, an enzyme or a chemical feedstock. The present invention provides relates to a composition which comprises cellulolytic and/or hemicellulolytic enzyme activity and which has the ability to modify, for example degrade, a non-starch carbohydrate material. A non-starch carbohydrate material is a material which comprises, consists of or substantially consists of one or more non-starch carbohydrates. Carbohydrate in this context includes all saccharides, for example polysaccharides, oligosaccharides, disaccharides or monosaccharides.

A composition as described herein typically modifies a non-starch carbohydrate material by chemically modification of such material. Chemical modification of the carbohydrate material may result in the degradation of such material, for example by hydrolysis, oxidation or other chemical modification such as by the action of a lyase.

A non-starch carbohydrate suitable for modification by a composition as described herein is lignocellulose. The major polysaccharides comprising different lignocellulosic residues, which may be considered as a potential renewable feedstock, are cellulose (glucans), hemicelluloses (xylans, heteroxylans and xyloglucans). In addition, some hemicellulose may be present as glucomannans, for example in wood-derived feedstocks. The enzymatic hydrolysis of these polysaccharides to soluble sugars, including both monomers and multimers, for example glucose, cellobiose, xylose, arabinose, galactose, fructose, mannose, rhamnose, ribose, galacturonic acid, glucoronic acid and other hexoses and pentoses occurs under the action of different enzymes acting in concert.

In addition, pectins and other pectic substances such as arabinans may make up considerably proportion of the dry mass of typically cell walls from non-woody plant tissues (about a quarter to half of dry mass may be pectins).

Cellulose is a linear polysaccharide composed of glucose residues linked by $\beta$-1,4 bonds. The linear nature of the cellulose fibers, as well as the stoichiometry of the $\beta$-linked glucose (relative to $\alpha$) generates structures more prone to interstrand hydrogen bonding than the highly branched $\alpha$-linked structure of starch. Thus, cellulose polymers are generally less soluble, and form more tightly bound fibers than the fibers found in starch.

Endoglucanases (EG) and exo-cellobiohydrolases (CBH) catalyze the hydrolysis of insoluble cellulose to cellooligosaccharides (cellobiose as a main product), while $\beta$-glucosidases (BG) convert the oligosaccharides, mainly cellobiose and cellotriose into glucose.

Hemicellulose is a complex polymer, and its composition often varies widely from organism to organism, and from one tissue type to another. In general, a main component of hemicellulose is $\beta$-1,4-linked xylose, a five carbon sugar. However, this xylose is often branched at 0-3 and/or 0-2 atom of xylose, and can be substituted with linkages to arabinose, galactose, mannose, glucuronic acid, galacturonic acid or by esterification to acetic acid (and esterification of ferulic acid to arabinose). Hemicellulose can also contain glucan, which is a general term for $\beta$-linked six carbon sugars (such as the $\beta$-(1,3)(1,4 glucans and heteroglucans mentioned previously) and additionally glucomannans (in which both glucose and mannose are present in the linear backbone, linked to each other by $\beta$-linkages).

Xylanases together with other accessory enzymes, for example $\alpha$-L-arabinofuranosidases, feruloyl and acetylxylan esterases, glucuronidases, and $\beta$-xylosidases) catalyze the hydrolysis of hemicelluloses.

Pectic substances include pectins, arabinans, galactans and arabinogalactans. Pectins are the most complex polysaccharides in the plant cell wall. They are built up around a core chain of $\alpha$(1,4)-linked D-galacturonic acid units interspersed to some degree with L-rhamnose. In any one cell wall there are a number of structural units that fit this description and it has generally been considered that in a single pectic molecule, the core chains of different structural units are continuous with one another.

The principal types of structural unit are: galacturonan (homogalacturonan), which may be substituted with methanol on the carboxyl group and acetate on O-2 and O-3; rhamnogalacturonan I (RGI), in which galacturonic acid units alternate with rhamnose units carrying (1,4)-linked galactan and (1,5)-linked arabinan side-chains. The arabinan side-chains may be attached directly to rhamnose or indirectly through the galactan chains; xylogalacturonan, with single xylosyl units on O-3 of galacturonic acid (closely associated with RGI); and rhamnogalacturonan II (RGII), a particularly complex minor unit containing unusual sugars, for example apiose. An RGII unit may contain two apiosyl residues which, under suitable ionic conditions, can reversibly form esters with borate.

An enzyme composition will comprise enzymatic activities typically derived from a saprophyte fungal microorganism of the class Penicillium and from the genus *Talaromyces,* for example *Talaromyces emersonii. Talaromyces emersonii* may also be referred to as *Geosmithia emersonii* or *Penicillium emersonii. Talaromyces emersonii* has also been referred to as *Talaromyces duponti* and *Penicillium duponti.*

An enzyme composition comprises at least two activities, although typically a composition will comprise more than two activities, for example, three, four, five, six, seven, eight, nine or more. Typically, an enzyme composition of the invention may comprise at least one cellulase and at least one hemicellulase. However, an enzyme composition of the invention may comprise cellulases, but no xylanases. In addition, a enzyme composition of the invention may comprise auxiliary enzyme activity, i.e. additional activity which, either directly or indirectly leads to lignocellulose degradation. Examples of such auxiliary activities are mentioned herein.

Thus, the enzyme composition may comprise endoglucanase activity and/or cellobiohydrolase activity and/or β-glucosidase activity. The enzyme composition may comprise more than one enzyme activity in one or more of those classes. For example, the enzyme composition may comprise two endoglucanase activities, for example, endo-1,3(1,4)-β glucanase activity and endo-β-1,4-glucanase activity. Such a composition may also comprise one or more xylanase activities. Such a composition may comprise an auxiliary enzyme activity.

The enzyme composition may be derived from *Talaromyces emersonii.* In the invention, it is anticipated that a core set of (lignocellulose degrading) enzyme activities may be derived from *Talaromyces emersonii. Talaromyces emersonii* can provide a highly effective set of activities as demonstrated herein for the hydrolysis of lignocellulosic biomass. That activity can then be supplemented with additional enzyme activities from other sources. Such additional activities may be derived from classical sources and/or produced by a genetically modified organism.

The activities in the enzyme composition may be thermostable. Herein, this means that the activity has a temperature optimum of 40°C or higher, for example about 50°C or higher, such as about 60°C or higher, for example about 70°C or higher, such as about 75°C or higher, for example about 80°C or higher such as 85°C or higher. Activities in the enzyme composition will typically not have the same temperature optima, but preferably will, nevertheless, be thermostable.

In addition, enzyme activities in the enzyme composition may be able to work at low pH. For the purposes of this invention, low pH indicates a pH of about 5.5 or lower, about 5 or lower, about 4.5 or lower, about 4.0 or lower or about 3.8 or lower or about 3.5 or lower.

Activities in the enzyme composition may be defined by a combination of any of the above temperature optima and pH values.

The composition used in a method of the invention may comprise, in addition to the activities derived from *Talaroymces,* a cellulase (for example one derived from a source other than *Talaromyces*) and/or a hemicellulase (for example one derived from a source other than *Talaromyces*) and/or a pectinase.

The enzyme composition may comprise one, two or three classes of cellulase, for example one, two or all of an endoglucanase (EG), an exo-cellobiohydrolase (CBH) and a β- glucosidase (BG). The enzyme composition may comprise two or more of any of these classes of cellulase.

An enzyme composition of the invention may comprise an activity which has a different type of cellulase activity and/or hemicellulase activity and/or pectinase activity than that provided by the enzyme composition. For example, an enzyme composition of the invention may comprise one type of cellulase and/or hemicellulase activity and/or pectinase activity provided by a composition as described herein and a second type of cellulase and/or hemicellulase activity and/or pectinase activity provided by an additional cellulose/hemicellulase/pectinase.

Herein, a cellulase is any polypeptide which is capable of degrading or modifying cellulose. A polypeptide which is capable of degrading cellulose is one which is capable of catalysing the process of breaking down cellulose into smaller units, either partially, for example into cellodextrins, or completely into glucose monomers. A cellulase according to the invention may give rise to a mixed population of cellodextrins and glucose monomers when contacted with the cellulase. Such degradation will typically take place by way of a hydrolysis reaction.

Herein, a hemicellulase is any polypeptide which is capable of degrading or modifying hemicellulose. That is to say, a hemicellulase may be capable of degrading or modifying one or more of xylan, glucuronoxylan, arabinoxylan, glucomannan and xyloglucan. A polypeptide which is capable of degrading a hemicellulose is one which is capable of catalysing the process of breaking down the hemicellulose into smaller polysaccharides, either partially, for example into oligosaccharides, or completely into sugar monomers, for example hexose or pentose sugar monomers. A hemicellulase according to the invention may give rise to a mixed population of oligosaccharides and sugar monomers when contacted with the hemicellulase. Such degradation will typically take place by way of a hydrolysis reaction.

Herein, a pectinase is any polypeptide which is capable of degrading or modifying pectin. A polypeptide which is capable of degrading pectin is one which is capable of catalysing the process of breaking down pectin into smaller units, either partially, for example into oligosaccharides, or completely into sugar monomers. A pectinase according to the invention may give rise to a mixed population of oligosacchardies and sugar monomers when contacted with the pectinase. Such degradation will typically take place by way of a hydrolysis reaction.

Accordingly, an enzyme composition of the invention may comprise any cellulase, for example, a cellobiohydrolase, an endo-β-1,4-glucanase, a β-glucosidase or a β-(1,3)(1,4)-glucanase.

Herein, a cellobiohydrolase (EC 3.2.1.91) is any polypeptide which is capable of catalysing the hydrolysis of 1,4-β-D-glucosidic linkages in cellulose or cellotetraose, releasing cellobiose from the ends of the chains. This enzyme may also be referred to as cellulase 1,4-β-cellobiosidase, 1,4-β-cellobiohydrolase, 1,4-β-D-glucan cellobiohydrolase, avicelase, exo-1,4-β-D-glucanase, exocellobiohydrolase or exoglucanase. Cellobiohydrolases may be subdivided into cellobiohydrolase I (CBH I) and cellobiohydrolase II (CBH II). CBH I is defined as cellobiohydrolases that hydrolyse cellulose predominantly from the reducing ends, splitting off cellobiose. CBH II is defined as cellobiohydrolases that hydrolyse cellulose from predominantly from the non-reducing ends, splitting of cellobiose.

Herein, an endo-β-1,4-glucanase (EC 3.2.1.4) is any polypeptide which is capably of catalysing the endohydrolysis of 1,4-β-D-glucosidic linkages in cellulose, lichenin or cereal β-D-glucans. Such a polypeptide may also be capable of hydrolyzing 1,4-linkages in β-D-glucans also containing 1,3-linkages. This enzyme may also be referred to as cellulase, avicelase, β-1,4-endoglucan hydrolase, β-1,4-glucanase, carboxymethyl cellulase, celludextrinase, endo-1,4-β-D-glucanase, endo-1,4-β-D-glucanohydrolase, endo-1,4-β-glucanase or endoglucanase. CEA is herein an endoglucanase EC 3.2.1.4 that based on its 3D structure is classified under Glycosyl Hydrolase familiy 5 (GH5). Known activities for GH5 include chitosanase (EC 3.2.1.132); β-mannosidase (EC 3.2.1.25); Cellulase (EC 3.2.1.4); glucan 1,3-β-glucosidase (EC 3.2.1.58); licheninase (EC 3.2.1.73); glucan endo-1,6-β-glucosidase (EC 3.2.1.75); mannan endo-β-1,4-mannosidase (EC 3.2.1.78); endo-β-1,4-xylanase (EC 3.2.1.8); cellulose β-1,4-cellobiosidase (EC 3.2.1.91); endo-β-1,6-galactanase (EC 3.2.1.-): β-1,3-mannanase (EC 3.2.1.-); xyloglucan-specific endo-β-1,4-glucanase (EC 3.2.1.151); mannan transglycosylase (EC 2.4.1.-). CEB is herein an endoglucanase EC 3.2.1.4 that based on its 3D structure is classified under Glycosyl Hydrolase familiy 7 (GH7). Known activities for GH7 include endo-β-1,4-glucanase (EC 3.2.1.4); [reducing end-acting] cellobiohydrolase (EC 3.2.1.-); chitosanase (EC 3.2.1.132); endo-β-1,3-1,4-glucanase (EC 3.2.1.73)

Herein, a β-glucosidase, abbreviated BG (EC 3.2.1.21) is any polypeptide which is capable of catalysing the hydrolysis of terminal, non-reducing β-D-glucose residues with release of β-D-glucose. Such a polypeptide may have a wide specificity for β-D-glucosides and may also hydrolyze one or more of the following: a β-D-galactoside, an α-L-arabinoside, a β-D-xyloside or a β-D-fucoside. This enzyme may also be referred to as amygdalase, β-D-glucoside glucohydrolase, cellobiase or gentobiase.

Herein a β-(1,3)(1,4)-glucanase (EC 3.2.1.73) is any polypeptide which is capable of catalyzing the hydrolysis of 1,4-β-D-glucosidic linkages in β-D-glucans containing 1,3- and 1,4-bonds. Such a polypeptide may act on lichenin and cereal β-D-glucans, but not on β-D-glucans containing only 1,3- or 1,4-bonds. This enzyme may also be referred to as licheninase, 1,3-1,4-β-D-glucan 4-glucanohydrolase, β-glucanase, endo-β-1,3-1,4 glucanase, lichenase or mixed linkage β-glucanase. An alternative for this type of enzyme is EC 3.2.1.6, which is described as endo-1,3(4)-beta-glucanase. This type of enzyme hydrolyses 1,3- or 1,4-linkages in beta-D-glucans when the glucose residue whose reducing group is involved in the linkage to be hydrolysed is itself substituted at C-3. Alternative names include endo-1,3-beta-glucanase, laminarinase, 1,3-(1,3;1,4)-beta-D-glucan 3 (4) glucanohydrolase; substrates include laminarin, lichenin and cereal beta-D-glucans.

An enzyme composition of the invention may comprise any hemicellulase, for example, an endoxylanase, a β-xylosidase, a α-L-arabionofuranosidase, an α-D-glucuronidase, an acetyl xylan esterase, a feruloyl esterase, a coumaroyl esterase, an α-galactosidase, a β-galactosidase, a β-mannanase or a β-mannosidase.

Herein, an endoxylanase (EC 3.2.1.8) is any polypeptide which is capable of catalyzing the endohydrolysis of 1,4-β-D-xylosidic linkages in xylans. This enzyme may also be referred to as endo-1,4-β-xylanase or 1,4-β-D-xylan xylanohydrolase. An alternative is EC 3.2.1.136, a glucuronoarabinoxylan endoxylanase, an enzyme that is able to hydrolyse 1,4 xylosidic linkages in glucuronoarabinoxylans.

Herein, a β-xylosidase (EC 3.2.1.37) is any polypeptide which is capable of catalyzing the hydrolysis of 1,4-β-D-xylans, to remove successive D-xylose residues from the non-reducing termini. Such enzymes may also hydrolyze xylobiose. This enzyme may also be referred to as xylan 1,4-β-xylosidase, 1,4-β-D-xylan xylohydrolase, exo-1,4-β-xylosidase or xylobiase.

Herein, an α-L-arabinofuranosidase (EC 3.2.1.55) is any polypeptide which is capable of acting on α-L-arabinofuranosides, α-L-arabinans containing (1,2) and/or (1,3)- and/or (1,5)-linkages, arabinoxylans and arabinogalactans. This enzyme may also be referred to as α-N-arabinofuranosidase, arabinofuranosidase or arabinosidase.

Herein, an α-D-glucuronidase (EC 3.2.1.139) is any polypeptide which is capable of catalyzing a reaction of the following form: alpha-D-glucuronoside + H(2)O = an alcohol + D-glucuronate. This enzyme may also be referred to as alpha-glucuronidase or alpha-glucosiduronase. These enzymes may also hydrolyse 4-O-methylated glucoronic acid, which can also be present as a substituent in xylans. Alternative is EC 3.2.1.131: xylan alpha-1,2-glucuronosidase, which catalyses the hydrolysis of alpha-1,2-(4-O-methyl)glucuronosyl links.

Herein, an acetyl xylan esterase (EC 3.1.1.72) is any polypeptide which is capable of catalyzing the deacetylation of xylans and xylo-oligosaccharides. Such a polypeptide may catalyze the hydrolysis of acetyl groups from polymeric xylan, acetylated xylose, acetylated glucose, alpha-napthyl acetate or p-nitrophenyl acetate but, typically, not from triacetylglycerol. Such a polypeptide typically does not act on acetylated mannan or pectin.

Herein, a feruloyl esterase (EC 3.1.1.73) is any polypeptide which is capable of catalyzing a reaction of the form: feruloyl-saccharide + H(2)O = ferulate + saccharide. The saccharide may be, for example, an oligosaccharide or a polysaccharide. It may typically catalyze the hydrolysis of the 4-hydroxy-3-methoxycinnamoyl (feruloyl) group from an esterified sugar, which is usually arabinose in 'natural' substrates. p-nitrophenol acetate and methyl ferulate are typically poorer substrates. This enzyme may also be referred to as cinnamoyl ester hydrolase, ferulic acid esterase or hydroxycinnamoyl esterase. It may also be referred to as a hemicellulase accessory enzyme, since it may help xylanases and pectinases to break down plant cell wall hemicellulose and pectin.

Herein, a coumaroyl esterase (EC 3.1.1.73) is any polypeptide which is capable of catalyzing a reaction of the form: coumaroyl-saccharide + H(2)O = coumarate + saccharide. The saccharide may be, for example, an oligosaccharide or a polysaccharide. This enzyme may also be referred to as trans-4-coumaroyl esterase, trans-p-coumaroyl esterase, p-coumaroyl esterase or p-coumaric acid esterase. This enzyme also falls within EC 3.1.1.73 so may also be referred to as a feruloyl esterase.

Herein, an $\alpha$-galactosidase (EC 3.2.1.22) is any polypeptide which is capable of catalyzing the hydrolysis of of terminal, non-reducing $\alpha$-D-galactose residues in $\alpha$-D-galactosides, including galactose oligosaccharides, galactomannans, galactans and arabinogalactans. Such a polypeptide may also be capable of hydrolyzing $\alpha$-D-fucosides. This enzyme may also be referred to as melibiase.

Herein, a $\beta$-galactosidase (EC 3.2.1.23) is any polypeptide which is capable of catalyzing the hydrolysis of terminal non-reducing $\beta$-D-galactose residues in $\beta$-D-galactosides. Such a polypeptide may also be capable of hydrolyzing $\alpha$-L-arabinosides. This enzyme may also be referred to as exo-(1->4)-$\beta$-D-galactanase or lactase.

Herein, a $\beta$-mannanase (EC 3.2.1.78) is any polypeptide which is capable of catalyzing the random hydrolysis of 1,4-$\beta$-D-mannosidic linkages in mannans, galactomannans and glucomannans. This enzyme may also be referred to as mannan endo-1,4-$\beta$-mannosidase or endo-1,4-mannanase.

Herein, a $\beta$-mannosidase (EC 3.2.1.25) is any polypeptide which is capable of catalyzing the hydrolysis of terminal, non-reducing $\beta$-D-mannose residues in $\beta$-D-mannosides. This enzyme may also be referred to as mannanase or mannase.

An enzyme composition of the invention may comprise any pectinase, for example an endo polygalacturonase, a pectin methyl esterase, an endo-galactanase, a beta galactosidase, a pectin acetyl esterase, an endo-pectin lyase, pectate lyase, alpha rhamnosidase, an exo-galacturonase, an expolygalacturonate lyase, a rhamnogalacturonan hydrolase, a rhamnogalacturonan lyase, a rhamnogalacturonan acetyl esterase, a rhamnogalacturonan galacturonohydrolase, a xylogalacturonase.

Herein, an endo-polygalacturonase (EC 3.2.1.15) is any polypeptide which is capable of catalyzing the random hydrolysis of 1,4-$\alpha$-D-galactosiduronic linkages in pectate and other galacturonans. This enzyme may also be referred to as polygalacturonase pectin depolymerase, pectinase, endopolygalacturonase, pectolase, pectin hydrolase, pectin polygalacturonase, poly-$\alpha$-1,4-galacturonide glycanohydrolase, endogalacturonase; endo-D-galacturonase or poly(1,4-$\alpha$-D-galacturonide) glycanohydrolase.

Herein, a pectin methyl esterase (EC 3.1.1.11) is any enzyme which is capable of catalyzing the reaction: pectin + n $H_2O$ = n methanol + pectate. The enzyme may also been known as pectinesterase, pectin demethoxylase, pectin methoxylase, pectin methylesterase, pectase, pectinoesterase or pectin pectylhydrolase.

Herein, an endo-galactanase (EC 3.2.1.89) is any enzyme capable of catalyzing the endohydrolysis of 1,4-$\beta$-D-galactosidic linkages in arabinogalactans. The enzyme may also be known as arabinogalactan endo-1,4-$\beta$-galactosidase, endo-1,4-$\beta$-galactanase, galactanase, arabinogalactanase or arabinogalactan 4-$\beta$-D-galactanohydrolase.

Herein, a pectin acetyl esterase is defined herein as any enzyme which has an acetyl esterase activity which catalyzes the deacetylation of the acetyl groups at the hydroxyl groups of GalUA residues of pectin

Herein, an endo-pectin lyase (EC 4.2.2.10) is any enzyme capable of catalyzing the eliminative cleavage of (1 →4)-$\alpha$-D-galacturonan methyl ester to give oligosaccharides with 4-deoxy-6-*O*-methyl-$\alpha$-D-galact-4-enuronosyl groups at their non-reducing ends. The enzyme may also be known as pectin lyase, pectin *trans*-eliminase; endo-pectin lyase, polymethylgalacturonic transeliminase, pectin methyltranseliminase, pectolyase, PL, PNL or PMGL or (1→4)-6-*O*-methyl-$\alpha$-D-galacturonan lyase.

Herein, a pectate lyase (EC 4.2.2.2) is any enzyme capable of catalyzing the eliminative cleavage of (1 →4)-$\alpha$-D-galacturonan to give oligosaccharides with 4-deoxy-$\alpha$-D-galact-4-enuronosyl groups at their non-reducing ends. The enzyme may also be known polygalacturonic transeliminase, pectic acid transeliminase, polygalacturonate lyase, endopectin methyltranseliminase, pectate transeliminase, endogalacturonate transeliminase, pectic acid lyase, pectic lyase, $\alpha$-1,4-D-endopolygalacturonic acid lyase, PGA lyase, PPase-N, endo-$\alpha$-1,4-polygalacturonic acid lyase, polygalacturonic acid lyase, pectin trans-eliminase, polygalacturonic acid trans-eliminase or (1 →4)-$\alpha$-D-galacturonan lyase.

Herein, an alpha rhamnosidase (EC 3.2.1.40) is any polypeptide which is capable of catalyzing the hydrolysis of terminal non-reducing $\alpha$-L-rhamnose residues, in $\alpha$-L-rhamnosides or alternatively in rhamnogalacturonan. This enzyme may also be known as $\alpha$-L-rhamnosidase T, $\alpha$-L-rhamnosidase N or $\alpha$-L-rhamnoside rhamnohydrolase.

Herein, exo-galacturonase (EC 3.2.1.82) is any polypeptide capable of hydrolysis of pectic acid from the non-reducing end, releasing digalacturonate. The enzyme may also be known as exo-poly-$\alpha$-galacturonosidase, exopolyg-alacturonosidase or exopolygalacturanosidase.

Herein, exo-galacturonase (EC 3.2.1.67) is any polypeptide capable of catalyzing: $(1,4$-$\alpha$-D-galacturonide$)_n$ + $H_2O$ = $(1,4$-$\alpha$-D-galacturonide$)_{n-1}$ + D-galacturonate. The enzyme may also be known as galacturan 1,4-$\alpha$-galacturoni-dase, exopolygalacturonase, poly(galacturonate) hydrolase, exo-D-galacturonase, exo-D-galacturonanase, exopoly-D-galacturonase or poly(1,4-$\alpha$-D-galacturonide) galacturonohydrolase.

Herein, exopolygalacturonate lyase (EC 4.2.2.9) is any polypeptide capable of catalyzing eliminative cleavage of 4-(4-deoxy-$\alpha$-D-galact-4-enuronosyl)-D-galacturonate from the reducing end of pectate, i.e. de-esterified pectin. This enzyme may be known as pectate disaccharide-lyase, pectate exo-lyase, exopectic acid transeliminase, exopectate lyase, exopolygalacturonic acid-*trans*-eliminase, PATE, exo-PATE, exo-PGL or $(1 \to 4)$-$\alpha$-D-galacturonan reducing-end-disaccharide-lyase.

Herein, rhamnogalacturonan hydrolase is any polypeptide which is capable of hydrolyzing the linkage between galactosyluronic acid acid and rhamnopyranosyl in an endo-fashion in strictly alternating rhamnogalacturonan structures, consisting of the disaccharide [(1,2-alpha-L-rhamnoyl-(1,4)-alpha-galactosyluronic acid].

Herein, rhamnogalacturonan lyase is any polypeptide which is any polypeptide, which is capable of cleaving $\alpha$-L-Rha*p*-$(1 \to 4)$-$\alpha$-D-Ga*p*A linkages in an endo-fashion in rhamnogalacturonan by beta-elimination.

Herein, rhamnogalacturonan acetyl esterase is any polypeptide which catalyzes the deacetylation of the backbone of alternating rhamnose and galacturonic acid residues in rhamnogalacturonan.

Herein, rhamnogalacturonan galacturonohydrolase is any polypeptide which is capable of hydrolyzing galacturonic acid from the non-reducing end of strictly alternating rhamnogalacturonan structures in an exo-fashion.

Herein, xylogalacturonase is any polypeptide which acts on xylogalacturonan by cleaving the $\beta$-xylose substituted galacturonic acid backbone in an *endo*-manner. This enzyme may also be known as xylogalacturonan hydrolase.

Herein, an $\alpha$-L-arabinofuranosidase (EC 3.2.1.55) is any polypeptide which is capable of acting on $\alpha$-L-arab-inofuranosides, $\alpha$-L-arabinans containing (1,2) and/or (1,3)- and/or (1,5)-linkages, arabinoxylans and arabinogalactans. This enzyme may also be referred to as $\alpha$-N-arabinofuranosidase, arabinofuranosidase or arabinosidase.

Herein, endo-arabinanase (EC 3.2.1.99) is any polypeptide which is capable of catalyzing endohydrolysis of 1,5-$\alpha$-arabinofuranosidic linkages in 1,5-arabinans. The enzyme may also be know as endo-arabinase, arabinan endo-1,5-$\alpha$-L-arabinosidase, endo-1,5-$\alpha$-L-arabinanase, endo-$\alpha$-1,5-arabanase; endo-arabanase or 1,5-$\alpha$-L-arabinan 1,5-$\alpha$-L-arabinanohydrolase.

An enzyme composition of the invention may comprise at least one cellulase and/or at least one hemicellulase and/or at least one pectinase (one of which is a polypeptide according to the invention). An enzyme composition of the invention may comprise a cellobiohydrolase, an endoglucanase and/or a $\beta$-glucosidase. Such a composition may also comprise one or more hemicellulases and/or one or more pectinases.

In addition, one or more (for example two, three, four or all) of an amylase, a protease, a lipase, a ligninase, a hexosyltransferase, a glucuronidase or an expansin or a cellulose induced protein or a cellulose integrating protein or like protein may be present in an enzyme composition of the invention (these are referred to as auxiliary activities above).

"Protease" includes enzymes that hydrolyze peptide bonds (peptidases), as well as enzymes that hydrolyze bonds between peptides and other moieties, such as sugars (glycopeptidases). Many proteases are characterized under EC 3.4, and are suitable for use in the invention incorporated herein by reference. Some specific types of proteases include, cysteine proteases including pepsin, papain and serine proteases including chymotrypsins, carboxypeptidases and metalloendopeptidases.

"Lipase" includes enzymes that hydrolyze lipids, fatty acids, and acylglycerides, including phospoglycerides, lipoproteins, diacylglycerols, and the like. In plants, lipids are used as structural components to limit water loss and pathogen infection. These lipids include waxes derived from fatty acids, as well as cutin and suberin.

"Ligninase" includes enzymes that can hydrolyze or break down the structure of lignin polymers. Enzymes that can break down lignin include lignin peroxidases, manganese peroxidases, laccases and feruloyl esterases, and other enzymes described in the art known to depolymerize or otherwise break lignin polymers. Also included are enzymes capable of hydrolyzing bonds formed between hemicellulosic sugars (notably arabinose) and lignin. Ligninases include but are not limited to the following group of enzymes: lignin peroxidases (EC 1.11.1.14), manganese peroxidases (EC 1.11.1.13), laccases (EC 1.10.3.2) and feruloyl esterases (EC 3.1.1.73).

"Hexosyltransferase" (2.4.1-) includes enzymes which are capable of catalyzing a transferase reaction, but which can also catalyze a hydrolysis reaction, for example of cellulose and/or cellulose degradation products. An example of a hexosyltransferase which may be used in the invention is a $\beta$-glucanosyltransferase. Such an enzyme may be able to catalyze degradation of (1,3)(1,4)glucan and/or cellulose and/or a cellulose degradation product.

"Glucuronidase" includes enzymes that catalyze the hydrolysis of a glucoronoside, for example $\beta$-glucuronoside to yield an alcohol. Many glucuronidases have been characterized and may be suitable for use in the invention, for example β-glucuronidase (EC 3.2.1.31), hyalurono-glucuronidase (EC 3.2.1.36), glucuronosyl-disulfoglucosamine glucuronidase (3.2.1.56), glycyrrhizinate β-glucuronidase (3.2.1.128) or a-D-glucuronidase (EC 3.2.1.139).

The enzyme composition may comprise an expansin or expansin-like protein, such as a swollenin (see Salheimo et al., Eur. J. Biohem. 269, 4202-4211, 2002) or a swollenin-like protein.

Expansins are implicated in loosening of the cell wall structure during plant cell growth. Expansins have been proposed to disrupt hydrogen bonding between cellulose and other cell wall polysaccharides without having hydrolytic activity. In this way, they are thought to allow the sliding of cellulose fibers and enlargement of the cell wall. Swollenin, an expansin-like protein contains an N-terminal Carbohydrate Binding Module Family 1 domain (CBD) and a C-terminal expansin-like domain. For the purposes of this invention, an expansin-like protein or swollenin-like protein may comprise one or both of such domains and/or may disrupt the structure of cell walls (such as disrupting cellulose structure), optionally without producing detectable amounts of reducing sugars.

The enzyme composition may comprise a cellulose induced protein, for example the polypeptide product of the *cip1* or *cip2* gene or similar genes (see Foreman et al., J. Biol. Chem. 278(34), 31988-31997, 2003), a cellulose/cellulosome integrating protein, for example the polypeptide product of the cipA or cipC gene, or a scaffoldin or a scaffoldin-like protein. Scaffoldins and cellulose integrating proteins are multifunctional integrating subunits which may organize cellulolytic subunits into a multi-enzyme complex. This is accomplished by the interaction of two complementary classes of domain, i.e. a cohesion domain on scaffoldin and a dockerin domain on each enzymatic unit. The scaffoldin subunit also bears a cellulose-binding module (CBM) that mediates attachment of the cellulosome to its substrate. A scaffoldin or cellulose integrating protein for the purposes of this invention may comprise one or both of such domains.

A enzyme composition may be composed of a member of each of the classes of enzymes mentioned above, several members of one enzyme class, or any combination of these enzymes classes or helper proteins (i.e. those proteins mentioned herein which do not have enzymatic activity *per se,* but do nevertheless assist in lignocellulosic degradation).

A enzyme composition may be composed of enzymes from (1) commercial suppliers; (2) cloned genes expressing enzymes; (3) complex broth (such as that resulting from growth of a microbial strain in media, wherein the strains secrete proteins and enzymes into the media; (4) cell lysates of strains grown as in (3); and/or (5) plant material expressing enzymes. Different enzymes in the enzyme composition of the invention may be obtained from different sources.

The enzymes can be produced either exogenously in microorganisms, yeasts, fungi, bacteria or plants, then isolated and added, for example, to lignocellulosic feedstock. Alternatively, the enzymes are produced, but not isolated, and crude cell mass fermentation broth, or plant material (such as corn stover or wheat straw), and the like may be added to, for example, the feedstock. Alternatively, the crude cell mass or enzyme production medium or plant material may be treated to prevent further microbial growth (for example, by heating or addition of antimicrobial agents), then added to, for example, a feedstock. These crude enzyme mixtures may include the organism producing the enzyme. Alternatively, the enzyme may be produced in a fermentation that uses feedstock (such as corn stover or wheat straw) to provide nutrition to an organism that produces an enzyme(s). In this manner, plants that produce the enzymes may themselves serve as a lignocellulosic feedstock and be added into lignocellulosic feedstock.

In the uses and methods described herein, the components of the compositions described above may be provided concomitantly (i.e. as a single composition *per se*) or separately or sequentially.

The invention thus relates to methods in which the composition described above are used and to uses of the composition in industrial processes.

In principle, an enzyme composition of the invention may be used in any process which requires the treatment of a material which comprises non-starch polysaccharide. Thus, a polypeptide or enzyme composition of the invention may be used in the treatment of non-starch polysaccharide material. Herein, non-starch polysaccharide material is a material which comprises or consists essential of one or, more typically, more than one non-starch polysaccharide.

Typically, plants and fungi and material derived therefrom comprise significant quantities of non-starch polysaccharide material. Accordingly, a polypeptide of the invention may be used in the treatment of a plant or fungal material or a material derived therefrom.

An important component of plant non-starch polysaccharide material is lignocellulose (also referred to herein as lignocellulolytic biomass). Lignocellulose is plant material that is composed of cellulose and hemicellulose and lignin. The carbohydrate polymers (cellulose and hemicelluloses) are tightly bound to the lignin by hydrogen and covalent bonds. Accordingly, a polypeptide of the invention may be used in the treatment of lignocellulolytic material. Herein, lignocellulolytic material is a material which comprises or consists essential of lignocellulose. Thus, in a method of the invention for the treatment of a non-starch polysaccharide, the non-starch polysaccharide may be a lignocellulosic material/biomass.

Accordingly, the invention provides a method of treating a non-starch polysaccharide in which the treatment comprises the degradation and/or modification of cellulose and/or hemicellulose.

Degradation in this context indicates that the treatment results in the generation of hydrolysis products of cellulose and/or hemicellulose and/or a pectic substance, i.e. saccharides of shorter length are present as result of the treatment than are present in a similar untreated non-starch polysaccharide. Thus, degradation in this context may result in the liberation of oligosaccharides and/or sugar monomers.

All plants and fungi contain non-starch polysaccharide as do virtually all plant- and fungal-derived polysaccharide materials. Accordingly, in a method of the invention for the treatment of a non-starch polysaccharide, said non-starch polysaccharide may be provided in the form of a plant or a plant derived material or a material comprising a plant or plant derived material, for example a plant pulp, a plant extract, a foodstuff or ingredient therefore, a fabric, a textile or an item of clothing.

The invention provides a method for producing a sugar from a lignocellosic material which method comprises contacting a composition as described herein with the lignocellulosic material.

Such a method allows free sugars (monomers) and/or oligosaccharides to be generated from lignocellulosic biomass. These methods involve converting lignocellulosic biomass to free sugars and small oligosaccharides with a polypeptide or enzyme composition of the invention.

The process of converting a complex carbohydrate such as lignocellulose into sugars preferably allows conversion into fermentable sugars. Such a process may be referred to as "saccharification." Accordingly, a method of the invention may result in the liberation of one or more hexose and/or pentose sugars, such as one or more of glucose, cellobiose, xylose, arabinose, galactose, galacturonic acid, glucuronic acid, mannose, rhamnose, sucrose and fructose.

Lignocellulolytic biomass suitable for use in the invention includes virgin biomass and/or non-virgin biomass such as agricultural biomass, commercial organics, construction and demolition debris, municipal solid waste, waste paper and yard waste. Common forms of biomass include trees, shrubs and grasses, wheat, wheat straw, sugar cane bagasse, switch grass, miscanthus, corn, corn stover, corn husks, corn cobs, canola stems, soybean stems, sweet sorghum, corn kernel including fiber from kernels, products and by-products from milling of grains such as corn, wheat and barley (including wet milling and dry milling) often called "bran or fibre" as well as municipal solid waste, waste paper and yard waste. The biomass can also be, but is not limited to, herbaceous material, agricultural residues, forestry residues, municipal solid wastes, waste paper, and pulp and paper mill residues. "Agricultural biomass" includes branches, bushes, canes, corn and corn husks, energy crops, forests, fruits, flowers, grains, grasses, herbaceous crops, leaves, bark, needles, logs, roots, saplings, short rotation woody crops, shrubs, switch grasses, trees, vegetables, fruit peels, vines, sugar beet pulp, wheat midlings, oat hulls, and hard and soft woods (not including woods with deleterious materials). In addition, agricultural biomass includes organic waste materials generated from agricultural processes including farming and forestry activities, specifically including forestry wood waste. Agricultural biomass may be any of the aforementioned singularly or in any combination or mixture thereof.

Apart from virgin biomass or feedstocks already processed in food and feed or paper and pulping industries, the biomass/feedstock may additionally be pretreated with heat, mechanical and/or chemical modification or any combination of such methods in order to enhance enzymatic degradation.

The fermentable sugars can be converted to useful value-added fermentation products, non-limiting examples of which include amino acids, vitamins, pharmaceuticals, animal feed supplements, specialty chemicals, chemical feedstocks, plastics, solvents, fuels, or other organic polymers, lactic acid, and ethanol, including fuel ethanol.

Specific value-added products that may be produced by the methods of the invention include, but not limited to, biofuels (including ethanol and butanol and biogas); lactic acid; a plastic; a specialty chemical; an organic acid, including citric acid, succinic acid, fumaric acid, itaconic acid and maleic acid; 3-hydoxy-propionic acid, acrylic acid; acetic acid; 1,3-propane-diol; ethylene, glycerol; a solvent; an animal feed supplement; a pharmaceutical, such as a β-lactam antibiotic or a cephalosporin; vitamins; an amino acid, such as lysine, methionine, tryptophan, threonine, and aspartic acid; an industrial enzyme, such as a protease, a cellulase, an amylase, a glucanase, a lactase, a lipase, a lyase, an oxidoreductases, a transferase or a xylanase; and a chemical feedstock.

The composition, nature of substitution, and degree of branching of hemicellulose is very different in dicotyledonous plants (dicots, i.e., plant whose seeds have two cotyledons or seed leaves such as lima beans, peanuts, almonds, peas, kidney beans) as compared to monocotyledonous plants (monocots; i.e., plants having a single cotyledon or seed leaf such as corn, wheat, rice, grasses, barley). In dicots, hemicellulose is comprised mainly of xyloglucans that are 1,4-β-linked glucose chains with 1,6-β-linked xylosyl side chains. In monocots, including most grain crops, the principal components of hemicellulose are heteroxylans. These are primarily comprised of 1,4-β-linked xylose backbone polymers with 1,3 -α linkages to arabinose, galactose, mannose and glucuronic acid or 4-O-methyl-glucuronic acid as well as xylose modified by ester-linked acetic acids. Also present are β glucan comprised of 1,3- and 1,4-β-linked glucosyl chains. In monocots, cellulose, heteroxylans and β-glucan may be present in roughly equal amounts, each comprising about 15-25% of the dry matter of cell walls. Also, different plants may comprise different amounts of, and different compositions of, pectic substances. For example, sugar beet contains about 19% pectin and about 21% arabinan on a dry weight basis.

Accordingly, an enzyme composition of the invention may be tailored in view of the particular feedstock which is to be used. That is to say, the spectrum of activities in an enzyme composition of the invention may vary depending on the feedstock in question.

Enzyme combinations or physical treatments can be administered concomitantly or sequentially. The enzymes can be produced either exogenously in microorganisms, yeasts, fungi, bacteria or plants, then isolated and added to the lignocellulosic feedstock. Alternatively, the enzymes are produced, but not isolated, and crude cell mass fermentation broth, or plant material (such as corn stover), and the like are added to the feedstock. Alternatively, the crude cell mass or enzyme production medium or plant material may be treated to prevent further microbial growth (for example, by heating or addition of antimicrobial agents), then added to the feedstock. These crude enzyme mixtures may include the organism producing the enzyme. Alternatively, the enzyme may be produced in a fermentation that uses feedstock (such as corn stover) to provide nutrition to an organism that produces an enzyme(s). In this manner, plants that produce the enzymes may serve as the lignocellulosic feedstock and be added into lignocellulosic feedstock.

In the method of the invention, a enzyme or combination of enzymes acts on a lignocellulosic substrate or plant biomass, serving as the feedstock, so as to convert this complex substrate to simple sugars and oligosaccharides for the production of ethanol or other useful fermentation products.

Accordingly, another aspect of the invention includes methods that utilize the composition described above together with further enzymes or physical treatments such as temperature and pH to convert the lignocellulosic plant biomass to sugars and oligosaccharides.

While the composition has been discussed as a single mixture it is recognized that the enzymes may be added sequentially where the temperature, pH, and other conditions may be altered to increase the activity of each individual enzyme. Alternatively, an optimum pH and temperature can be determined for the enzyme mixture.

The composition is reacted with substrate under any appropriate conditions. For example, enzymes can be incubated at about 25°C, about 30 °C, about 35 °C, about 37 °C, about 40 °C, about 45 °C, about 50 °C, about 55 °C, about 60°C, about 65°C, about 70°C, about 75°C, about 80°C, about 85°C, about 90°C or higher. That is, they can be incubated at a temperature of from about 20°C to about 95 °C, for example in buffers of low to medium ionic strength and/or from low to neutral pH. By "medium ionic strength" is intended that the buffer has an ion concentration of about 200 millimolar (mM) or less for any single ion component. The pH may range from about pH 2.5, about pH 3.0, about pH 3.5, about pH 4.0, about pH 4.5, about pH 5, about pH 5.5, about pH 6, about pH 6.5, about pH 7, about pH 7.5, about pH 8.0, to about pH 8.5. Generally, the pH range will be from about pH 3.0 to about pH 9.

Typically, the reaction may be carried out under low pH conditions as defined above. Thus, a method of the invention may be carried out such that no pH adjustment (i.e. to a more neutral pH is required). That is to say, an acid pretreated feedstock may be used as is with no requirement to addition of, for example, sodium hydroxide, prior to addition of an enzyme composition of the invention.

The feedstock may be washed prior to liquefaction/hydrolysis. Such washing may be with, for example, water.

Incubation of a composition under these conditions results in release or liberation of substantial amounts of the sugar from the lignocellulosic material. By substantial amount is intended at least about 20%, at least about 30%, at least about 40%, at least about 50%, at least about 60%, at least about 70%, at least about 80%, at least about 90%, at least about 95% or more of available sugar.

A liquefaction/hydrolysis or presaccharification step involving incubation with an enzyme or enzyme mixture can be utilized. This step can be performed at many different temperatures but it is preferred that the presaccharification occur at the temperature best suited to the enzyme mix being tested, or the predicted enzyme optimum of the enzymes to be tested. The temperature of the presaccharification may range from about 10 °C to about 80 °C, about 20 °C to about 80 °C, about 30 °C to about 70 °C, about 40 °C to about 60 °C, about 37 °C to about 50 °C, preferably about 37 °C to about 80 °C, more preferably about 50 °C. In the absence of data on the temperature optimum, it is preferable to perform the presaccharification reactions at 37 °C first, then at a higher temperature such as 50 °C. The pH of the presaccharification mixture may range from about 2.0 to about 10.0, but is preferably about 3.0 to about 5.0. Again, it may not be necessary to adjust the pH prior to saccharification since the enzyme composition is typically suitable for use at low pH as defined herein.

The liquefaction/hydrolysis or presaccharification step reaction may occur from several minutes to several hours, such as from about 1 hour to about 120 hours, preferably from about 2 hours to about 48 hours, more preferably from about 2 to about 24 hours, most preferably for from about 2 to about 6 hours. The cellulase treatment may occur from several minutes to several hours, such as from about 6 hours to about 168 hours, preferably about 12 hours to about 96 hours, more preferably about 24 hours to about 72 hours, even more preferably from about 24 hours to about 48 hours.

Sugars released from biomass can be converted to useful fermentation products such a one of those including, but not limited to, amino acids, vitamins, pharmaceuticals, animal feed supplements, specialty chemicals, chemical feedstocks, plastics, and ethanol, including fuel ethanol.

Significantly, a method of the invention may be carried out using high levels of dry matter (of the lignocellulosic material) in the hydrolysis reaction. Thus, the invention may be carried out with a dry matter content of about 5% or higher, about 8% or higher, about 10% or higher, about 15% or higher, about 20% or higher, about 25% or higher, about 30% or higher, about 35% or higher or about 40% or higher.

Accordingly, the invention provides a method for the preparation of a fermentation product, which method comprises:

a. degrading lignocellulose using a method as described herein; and
b. fermenting the resulting material,
thereby to prepare a fermentation product.

Such a process may be carried out without any requirement to adjust the pH during the process. That is to say, the process is one which may be carried out without the addition of any acid(s) or base(s). However, this excludes a pretreatment step, where acid may be added. The point is that the enzyme composition of the invention is capable of acting at low pH and, therefore, there is no need to adjust the pH of acid of an acid pretreated feedstock in order that saccharification may take place. Accordingly, a method of the invention may be a zero waste method using only organic products with no requirement for inorganic chemical input.

Fermentation products which may be produced according to the invention include amino acids, vitamins, pharmaceuticals, animal feed supplements, specialty chemicals, chemical feedstocks, plastics, solvents, fuels, or other organic polymers, lactic acid, and ethanol, including fuel ethanol (the term "ethanol" being understood to include ethyl alcohol or mixtures of ethyl alcohol and water).

Specific value-added products that may be produced by the methods of the invention include, but not limited to, biofuels (including ethanol and butanol); lactic acid; 3-hydroxy-propionic acid; acrylic acid; acetic acid; 1,3-propane-diol; ethylene; glycerol; a plastic; a specialty chemical; an organic acid, including citric acid, succinic acid and maleic acid; a solvent; an animal feed supplement; a pharmaceutical such as a β-lactam antibiotic or a cephalosporin; a vitamin; an amino acid, such as lysine, methionine, tryptophan, threonine, and aspartic acid; an enzyme, such as a protease, a cellulase, an amylase, a glucanase, a lactase, a lipase, a lyase, an oxidoreductase, a transferase or a xylanase; a chemical feedstock; or an animal feed supplement.

A method for the preparation of a fermentation product may optionally comprise recovery of the fermentation product.

Such a process may be carried out under aerobic or anaerobic conditions. Preferably, the process is carried out under micro-aerophilic or oxygen limited conditions and/or C-limited conditions.

An anaerobic fermentation process is herein defined as a fermentation process run in the absence of oxygen or in which substantially no oxygen is consumed, preferably about 5 or less, about 2.5 or less or about 1 mmol/L/h or less, and wherein organic molecules serve as both electron donor and electron acceptors.

An oxygen-limited fermentation process is a process in which the oxygen consumption is limited by the oxygen transfer from the gas to the liquid. The degree of oxygen limitation is determined by the amount and composition of the ingoing gasflow as well as the actual mixing/mass transfer properties of the fermentation equipment used. Preferably, in a process under oxygen-limited conditions, the rate of oxygen consumption is at least about 5.5, more preferably at least about 6 and even more preferably at least about 7 mmol/L/h.

The present invention also relates to mutants of *Talaromyces emersonii* that are capable of overproducing lignocelluloses-degrading enzymes that can degrade plant biomass. The microorganism is modified such that the synthesis of lignocelluloses enzymes is increased compared to the non-modified form of the microorganism.

The invention encompasses any mutation to a gene of the microorganism resulting in an increased production of lignocelluloses-degrading enzymes. Classical mutagenesis and a subsequent screening program is a well-known empirical, non-recombinant strain improvement approach (Vinci and Byng, 1999).

"Mutagenesis" means the induction of inheritable changes - mutations - into the genetic material of a microorganism, normally chromosomal DNA (Crueger, 1993). These mutations are caused by changes in the genotype of the microorganism (mutant). Mutations occur *in vivo* spontaneously or after induction by mutagens (e.g. radiation or chemical agents). Mutations can also be induced *in vitro* by the use of site-specific genetic engineering techniques.

Mutagenesis induced in microorganisms by physical or chemical agents occur at random. The most commonly used physical mutagens are heat, freezing and thawing, far ultraviolet radiation, near ultraviolet radiation in the presence of 8-methoxypsoralen and ionizing radiation. Preferred physical mutagen is UV irradiation.

The most commonly used chemical mutagens are: (i) mutagens affecting non-replicating DNA (e.g. nitrous acid, hydroxylamine and alkylating agents); and (ii) mutagens affecting replicating DNA (e.g. base analogs such as 5-bromouracil, 2-aminopurine and $N^4$-hydroxycytidine; and frameshift mutagens such as acridine orange, proflavine, 9-aminoacridine, acriflavine, ICR compounds ICR170 and ICR191, homidium bromide and photoreactive psoralens). Except for the ultraviolet radiation, alkylating agents, a group of chemical mutagens are the most potent mutagenic systems for practical application (Brookes, 1990, Crueger 1993). Most alkylating agents act as monofunctional agents such as epoxides (e.g. diepoxybutane [DEB]), ethyleneimines (e.g. ethyleneimine [EI]), alkyl sulfates (e.g. diethyl sulfate [DES], dimethyl sulfate [DMS], alkyl alkane-sulfonates (e.g. methyl methanesulfonate [MMS], ethyl methanesulfonate [EMS]), nitrosamides (e.g. methylnitrosourea [MNU], ethylnitrosourea [ENU], N-methyl-N'-nitro-N-nitrosoguanidine [NTG or MNNG], ethyl-N'-nitro-N-nitrosoguanidine [ENNG], propyl-N'-nitro-N-nitrosoguanidine [PNNG] 4-nitoquinoline [NQO]) and the indirect acting dimethyl nitrosamines [DMN] and diethyl nitrosamines [DEN]. Di- and polyfunctional alkylating agents are nitrogen mustards (e.g. di-(2-chloroethyl)sulfide [mustard gas], di-(2-chloroethyl)amine [nitrogen mustard], ICR compounds, mitomycin C). Preferred chemical mutagens are NTG, and NQO or combinations of the two.

For radiation or chemical agents, the mutational changes are usually the results of errors in replication or repair mechanisms by incorporating the wrong base or nucleotide into the polynucleotide chain, or by a direct chemical interaction with a base or nucleotide. Repair mechanisms are e.g. photo-reactivation, long-patch and base excision repair, post-replication repair, SOS repair; repair of alkylations, adaptive response and repair of DNA double-strand-breaks.

When using chemical or physical mutagens, typical survival percentages used in the art ranged between 0.01% and 60%. Preferably, the survival percentage is ranged between 0.05% and 50%. More preferably, the survival percentage is 0.1% and 30%. It is well known to the skilled person that conidiospores is the preferred material to mutagenize fungal microorganisms, including *Talaromyces* species. Mutants may however also be obtained from mycelium cells. The selection method described herein may be applied to select mutants obtained from either conidiospores or mycelium cells.

In addition to these techniques, mutations in mutator genes can also enhance mutagenesis in microorganisms by increasing the rate of spontaneous mutations in other genes. *E. coli* e.g., has at least nine loci that are known to cause mutator phenotypes: e.g. *mutT* locus increases mutation frequency about 1000 fold, mainly as an AT→CG transversion; *mutD5* is the most potent mutator, that has a defect of exonuclease from DNA polymerase III and a defect in the post-replicational DNA mismatch repair (Horiuchi et al., 1989; Schaaper and Radman, 1989; Crueger, 1993). Various mutator genes have also been genetically characterized in *B. subtilis.* Although the spontaneous mutation was not affected in the *mutT* mutant of *B. subtilis,* the frequency of spontaneous mutation can be enhanced greatly using the *mutS, mutL, mutM* and *mutY* mutants of *B. subtilis* (Ginetti *et al*., 1996; Sasaki *et al*., 2000; Sasaki and Kurusu, 2004).

A "mutation" may be a deletion, substitution or addition in the sequence of a gene. The mutation may be a disruption, frame shift mutation or nonsense mutation. The mutation may be a disruption affecting the entire sequence of the gene or a portion thereof. The mutation may affect the coding or the non-coding sequence, e.g., the promoter, of the gene. The mutation may result in complete lack of transcription of the gene or in premature termination of translation. Likewise, the mutation may be located in a preceding (or "upstream") gene, which disrupts transcription of the adjacent (or "downstream") gene(s) by a process referred to as polarity. Alternatively, the mutation may have the effect that the translated protein carries a mutation in comparison with the wild type protein that renders the protein non-functional.

Such mutations can increase cellulase production in a variety of ways. For example, mutations within the promoter and regulatory (cis) sites of a cellulose-encoding gene may directly affect expression of said gene thereby increasing the level or activity of the cellulase. Alternatively, mutations may affect the expression of genes that encode proteins involved in extracellular protein secretion mechanism. Increasing the level of these proteins may result in higher level of secretions into the culture medium. Also mutations may affect the proteases or RNA nucleases to prevent degradation of the mRNA transcript of the cellulase genes or the cellulase enzyme, thus increasing the levels of the cellulase protein relative to the unmutated cell.

Thus, the *Talaromyces* strains according to the invention may be prepared by a process that comprises the following steps:

a) Selecting and isolating one colony variant from a plurality of colony morphologies of a *Talaromyces* strain;
b) Subjecting the colony variant to mutagenesis, and screening of the resulting mutants for high cellulase activity;
c) Selecting and isolating from the mutants one or more high cellulase mutant strain;
and, optionally:
d) Selecting and isolating one colony variant of a high cellulase mutant strain from a plurality of colony morphologies of high cellulase mutant strain;
e) Subjecting strains from the colony variant of step d) to mutagenesis, and screening of the resulting mutants for resistance to glucose analogs (e.g. 2-deoxy-D-glucose [DOG]);
f) Selecting and isolating from the mutants from step e) one or more high cellulase glucose de-repressed mutant strain;
g) Subjecting the improved cellulase activity mutant from step c) again with mutagenesis, and screening of the resulting mutants for higher cellulase activity;
and optionally
h) Subjecting strains from step g) to mutagenesis, and screening of the resulting mutants for resistance to glucose analogs (e.g. 2-deoxy-D-glucose [DOG]);
i) Re-isolating a high cellulase glucose de-repressed mutant strain from the one or more high cellulase glucose de-repressed mutant strains from step h).

In one embodiment, in step b) the mutagen is NTG. NTG gives a high mutation factor for FOA (5 -fluoro-orotic acid) resistance.

In one embodiment, in steps e) and h) the mutagen is a combination of NQO and NTG. This combination of mutagens gives a high mutation factor for DOG (2-deoxy-D-glucose) resistance.

Efficiency of mutagenesis is herein estimated by determining survival after mutagenic treatment and the increase in the frequency of 5-fluoro orotic acid (FOA) resistant mutants, an easily scorable phenotype. The ratio of these frequencies is herein called the mutation factor (MF).

In one embodiment, the selecting of steps c) and f) is conducted in two steps, wherein the first screen is high-throughput screening and the second screen production of cellulases in shake flask cultures.

A "promoter" is a DNA sequence upstream from the start of transcription of a gene and involved in recognition and binding of RNA polymerase and/or other proteins to initiate transcription of the gene. Usually, the promoter determines under what conditions the gene is expressed.

The term "deregulated" or "deregulation" includes the alteration of modification of at least one gene in a microorganism such that the level or activity of the gene product in a microorganism is altered or modified. Preferably, at least one gene is altered or modified such that the gene product is enhanced or increased.

A "deregulated" promoter is one that permits gene expression under growth or environmental conditions where it is normally repressed. A "strong deregulated promoter" is one which causes mRNAs to be initiated at a higher frequency compared to a native promoter under growth or environmental conditions when it is de-repressed.

Mutations may decrease or completely prevent expression of one or more than one gene that represses the expression of cellulase-encoding genes. This would result in cells with increased cellulase production. Examples of such regulatory genes (also commonly referred to as transcription factors) are, but not limited to, *Trichoderma reesei* Acel and *Saccharomyces cerevisiae* mig1 and its orthologs found in fungi, including *Talaromyces emersonii* and related species.

Mutations may also increase expression or overexpression of one or more than one gene that activates expression of cellulose-encoding genes. This would result in cells with increased cellulase production. Examples of such regulatory genes (also commonly referred to as transcription factors) are, but not limited to, *Aspergillus niger* XlnR and *Trichoderma reesei* Acell and their orthologs found in fungi, including *Talaromyces emersonii* and related species.

Mutations may also increase expression of cellulase genes whose expression is repressed by glucose, a cellular state commonly referred to as glucose derepression or glucose derepressed mutations. Cells that are glucose derepressed for cellulase production are those that permit higher production of one or more cellulases when glucose is still present in the growth medium or in appreciable amounts in the cells where the cellulases are normally not produced or produced at lower levels. Glucose derepression can arise by various mutations in the cell, including but not limited to, mutations within the promoter region of a gene encoding cellulase which prevents binding of glucose-repressing regulatory proteins. Preventing binding of such regulatory proteins allows expression of the cellulase-encoded gene in the presence of glucose. An example of a regulatory protein that affects glucose repression of enzyme/cellulase production in fungi is, but not limited to, *Aspergillus nidulan* CreA and its orthologs found in fungi, including *Talaromyces emersonii* and related species. Alternatively, mutations in genes that regulate CreA and similar orthologs expression would also result in increased expression of the cellulase-encoded gene when glucose is present.

In one embodiment, the *Talaromyces* strain is glucose de-repressed. In one embodiment, the glucose de-repressed strain has a ratio of at least two, of enzyme production in the presence of glucose at 96 hours, relative to the enzyme production at similar conditions of the parental strain. In preferred embodiments the ratio at least 3, at least 4 at least 5, at least 6, at least 7, at least 8, at least 9, at least 10 at least 15, at least 20, at least 50, or at least 100. In one embodiment, the *Talaromyces* strain produces xylanase when grown in the presence of glucose.

In an embodiment, the glucose de-repressed strain has a cellulase activity that is at least 25% of the activity of the wild type (glucose repressed) parent strain when similarly grown in Talaromyces medium 2 without glucose for 96 hours.

In the present invention classical mutagenesis and screening was performed as follows: (i) Generation of mutant libraries using UV, or NTG alone or in a combination of with NQO; (ii) selection of random mutants for increased cellulase production or mutants that are resistant to glucose analogues that lead to glucose derepression of cellulase production; (iii) shake flask experiments of the mutants showing improved cellulase production in the presence or absence of glucose; and finally (iv) fermentation for determination of cellulase levels of the mutants that produce significantly more cellulase in shake flask experiments than the parent strain or produce cellulase in the presence of glucose.

The microorganism of the invention is capable of overproducing one or more lignocellulosic enzyme. As used herein, the term "overproducing a lignocellulosic enzyme" refers to significantly increased production of the lignocellulosic enzyme by the microorganism of the invention in comparison to the wild type form of said microorganism. Preferably, overproduction of lignocellulosic enzyme means production of at least 6 FPU per milliliter of culture medium.

Another aspect of the invention is a method for producing a cellulase mixture comprising (a) culturing a microorganism according to the invention under conditions such that the cellulase mixture is produced; and (b) optionally recovering the cellulase mixture from the cell culture medium.

The method of the invention comprises the step of culturing the modified microorganisms under conditions such that a cellulase mixture is produced. The term "culturing" includes maintaining and/or growing a living microorganism of the present invention. A microorganism of the invention is cultured in a nutrient medium suitable for production of the cellulase mixture using methods known in the art. The cultivation medium necessary for performing the cultivation process according to the invention is not critical to the invention. Nutrients are added to the process according to the needs of the organism in question, provided that the nutrients are supplied in excess. The cultivation medium conveniently contains a carbon source, a nitrogen source as well as additional compounds required for growth of the microorganism and/or the formation of the valuable cellulase mixture. For instance, additional compounds may be necessary for inducing the production of the valuable compound. The total amount of carbon and nitrogen source to be added to the cultivation process according to the invention may vary depending on e.g. the needs of the microorganism and/or the length of the cultivation process.

For example, a microorganism of the invention may be cultured in a suitable medium and under conditions allowing the cellulase mixture to be expressed and/or isolated. The culture takes place in a suitable nutrient medium comprising carbon and nitrogen sources and inorganic salts, using procedures known in the art (see, e. g., Bennett,J. W. and LaSure, L.,eds., More Gene Manipulations in Fungi, Academic Press, CA, 1991). Suitable media are available from commercial suppliers or may be prepared using published compositions (e. g., in catalogues of the American Type Culture Collection). Examples of suitable carbon sources or carbon substrates known in the art can be found in WO98/37179 include, but are not limited to, complex carbohydrates such as bean or grain meal, grain flour, starches, sugars, sugar alcohols, hydrocarbons, oils, fats, fatty acids, organic acids and alcohols. Grain flour is defined as, but not limited to milled rice, wheat, nude wheat, corn, barley, oat, or oatmeal.

Examples of suitable nitrogen sources known in the art include, but are not limited to, vegetable proteins, peptones, peptides and amino acids derived from grains, beans and tubers, proteins, peptides and amino acids derived from animal sources such as meat, milk and animal byproducts such as peptones, meat extracts, casein hydrolysates, soy bean meal, yeast extract, corn steep liquor, urea, ammonium sulfate, ammonium chloride, ammonium nitrate and ammonium phosphate.

Additional compounds required for growth of a microorganism and/or for product formation, like phosphate, sulphate, trace elements (e.g. magnesium, iron, manganese, calcium, copper, zinc, boron, molybdenum and/or cobalt salts), vitamins, or growth promoters may be added in amounts that may vary between different classes of microorganisms, i.e. between fungi, yeasts and bacteria. In addition, the amount of additional compound to be added may be determined by the type of valuable compound that is formed. Typically, the amount of medium components necessary for growth of a microorganism may be determined in relation to the amount of carbon source used in the cultivation, since the amount of biomass formed will be primarily determined by the amount of carbon source used.

The microorganism is preferably cultured on an inducer to induce cellulase enzymes. Inducers may be one or more oligo-, di- or mono-saccharide that leads to the induction of cellulase production by the host cell. The inducer may be produced by enzymatic hydrolysis of cellulose by one or more cellulase enzymes to produce mainly beta-linked glucose dimmers. Suitable inducers from commercial source include, but are not limited to, Avicell, Arbocell, Buckeye and Viscokraft. More preferably, is the isolation of a mutant of *Talaromyces* that does not require cellulose for induction of cellulase enzymes. The microorganisms may be cultured in liquid media either continuously or intermittently, by conventional culturing methods such as standing culture, test tube culture, shaking culture, aeration spinner culture or fermentation. Preferably, the microorganisms are cultured in a fermentor. A cultivation process according to the invention may be performed as a batch, a repeated batch, a fed-batch, a repeated fed-batch, a continuous cultivation, slurry or solid-state mode. For the specific application (i.e. in the examples) a batch process is applied where all medium components are added directly, as a whole in excess, to the medium before the start of the cultivation process. In a preferred embodiment, a glucose derepressed strain is cultured under fed-batch conditions. According to this embodiment very high enzyme titers may be produced, e.g. in fed-batch operation cellulose activities may be reached, e.g. of 20 FPU$_{2\%}$/ml or more, 25 FPU$_{2\%}$/ml or more or 30 FPU$_{2\%}$/ml or more. Fed-batch operation is not feasible with a glucose repressed strain, since the glucose that is fed in the fed-bacth operation will repress the cellulase production resulting in low cellulase yields.

Typically, a microbial cultivation process may be divided in a growth phase directed to formation of biomass and a production phase directed to production of a valuable compound. It is clear to the skilled person that these two phases may not be strictly separated in time, but may overlap to some extent. The cultivation process according to the invention is preferably performed on an industrial scale. An industrial scale process is understood to encompass a cultivation process on a fermenter volume scale which is $\geq 0.01$ m$^3$, preferably $\geq 0.1$ m$^3$, preferably $\geq 0.5$ m$^3$, preferably $\geq 5$ m$^3$, preferably $\geq 10$ m$^3$, more preferably $\geq 25$ m$^3$, most preferably $\geq 50$ m$^3$.

A further aspect of the present invention concerns the option of downstream processing of the cultivation broth. The batch process applied according to the invention facilitates downstream processing, especially because of the high yield of then valuable compound and the low amount of by-products. Downstream processing may include recovery as well as formulation steps.

After the cultivation process is ended, the valuable product may be recovered from the cultivation broth, using standard technology developed for recovery of the valuable compound of interest. The relevant downstream processing technology to be applied thereby depends on the nature and cellular localization of the valuable compound and on the desired purity level of the product of interest. In a typical recovery process, the biomass is separated from the cultivation fluid using e.g. centrifugation or filtration. The valuable compound then is recovered from the biomass in the case that the valuable product is accumulated inside or is associated with the microbial cells. The term "recovering" includes isolating, extracting, harvesting, separating or purifying the compound from culture media. Isolating the compound can be performed according to any conventional isolation or purification methodology known in the art including, but not limited to, alteration of pH, solvent extraction, dialysis, cell filtration and ultrafiltration, concentration, lyophilisation and the like.

Of course, the biomass as such may also be used. Otherwise, when the valuable product is excreted from the microbial cell, it is recovered from the cultivation fluid. The biomass and/or valuable compound may be formulated as liquid or solid products. In another embodiment, the desired compound is not purified from the microorganism or the culture. The entire culture or the culture supernatant may be used as a source of the product. In a specific embodiment, the culture or the culture supernatant is used without modification. In a further embodiment, the culture or the culture supernatant is added directly to lignocelluloses material for hydrolysis.

The various embodiments of the invention described herein may be cross-combined.

The following non-limiting examples further illustrate the invention.

## EXAMPLES

*General Methodology*

### *Strains*

*Talaromyces emersonii* strains of the present invention are derived from ATCC16479, which was formerly *Penicillium geosmithia emersonii.* Other designations of *T. emersonii* ATCC16479 are CBS393.64, IFO31232 and IMI116815. A single colony from ATCC16479 was purified and stored; this strain is TEC-1. A mass inoculation of TEC-1 was used to prepare stocks for further study, and this strain was named TEC-1A. All mutants described herein are derived from a single colony of TEC-1 A, named TEC-101.

### *Media and solutions*

1. General growth, colony purification, and strain conservation:

Potato dextrose agar, PDA, (Fluka, Cat. No. 70139)

| | |
|---|---|
| Potato extract | 4 g/l |
| Dextrose | 20 g/l |
| Bacto agar | 15 g/l |
| pH | 5.4 |
| Water | Adjust to one liter |
| Sterilize | 20 min at 120°C |

Physiological salts

| | |
|---|---|
| NaCl | 9 g/l |
| $Na_2HPO_4.2H_2O$ | 0.8 g/l |
| $KH_2PO_4$ | 0.14 g/l |
| pH | 7 (adjusted with 3.5M phosphoric acid) |

2. Sporulation:

*Talaromyces* agar medium

| | |
|---|---|
| Salt fraction no.3 | 15 g |
| Cellulose (3%) | 30 g |
| Bacto peptone | 7.5 g |
| Grain flour | 15 g |
| $KH_2PO_4$ | 5 g |
| CaCl2.2aq | 1 g |
| Bacto agar | 20 g |
| pH | 6.0 |
| Water | Adjust to one liter |
| Sterilize | 20 min at 120°C |

Salt fraction composition

The salt fraction was fitting the disclosure of WO98/37179, Table 1. Deviations from the composition of this table were CaCl2.2aq 1.0 g/l, KCl 1.8 g/L, citric acid 1 aq 0.45 g/L (chelating agent).

3. Fluorescence plate assay:

C agar

| | |
|---|---|
| 10x MS buffer | 100 ml |
| Bacto peptone | 1g |
| Cellulose (10%) | 100 ml |
| Oxoid agar no 3 | 20 g |
| Water | Adjust to one liter |
| Sterilize | 20 min at 120°C |

10x MS buffer

| | |
|---|---|
| $KH_2PO_4$ | 20 g |
| $(NH_4)_2SO_4$ | 13 g |
| Ureum | 3 g |
| $MgSO_4.7H_2O$ | 3 g |
| $CaCl_2$ | 3 g |
| $FeSO_4.7H_2O$ | 50 mg |
| $MnSO_4.H_2O$ | 16 mg |
| $ZnSO_4.7H_2O$ | 14 mg |
| Water | adjust to one liter 1 L |
| pH | 6.0 |
| Store | max 6 months, refrigerate |

4. Medium to reduce colony size:

CTX agar

C agar
Triton X100 (0.05%)

5. Germination of spores and growth stimulation:

CTXG agar

CTX agar
Glucose up to 1.5%

6. Selection of glucose analogue resistant mutants:

YNB (Yeast Nitrogen Base) agar:

| | |
|---|---|
| YNB + amino acids | 14 g |
| Starch soluble | 20 g |
| Bacto Peptone | 10 g |
| Cellulose | 10 g |
| Oxoid agar no 3 | 25 g |
| Water | adjust to one liter |
| pH | 6.2 |
| Sterilize | 20 min at 120°C |

YNB + amino acids (Difco, Cat. No. 239210)

7. Selection of Fluoro orotic acid resistant mutants:

FoA agar medium

| | |
|---|---|
| Bacto Yeast extract | 1 g |
| MES hydrate | 10 g |
| Glucose.$H_2O$ | 35 g |
| $(NH_4)_2SO4$ | 0.2 g |
| $KH_2PO4$ | 0.4 g |
| $NaH_2PO4$ | 0.15 g |
| $MgSO4.7H_2O$ | 0.5 g |
| $CaCl_2.2H_2O$ | 0.13 g |
| Tween-40 | 10 g |
| Uracil | 1 g |
| Oxoid agar no 1 | 30 g |
| Trace elements | 10 ml |
| Water | adjust to 1L |
| pH | adjust to 5.3 |
| Sterilize | 20 min, 120°C |

Filter sterilized fluoro orotic acid (Sigma, Cat. No. F5013; 10% stock solution prepared in DMSO) is added afterwards at a final concentration of 0.1 % (1 g/l).

*Shake flask media*

1. Inoculation medium:

Talaromyces medium 1

| Glucose | 20 g/L |
|---|---|
| Yeast extract (Difco) | 20 g/L |
| Clerol FBA3107 (AF) | 4 drops/L |
| pH | 6.0 |
| Sterilize | 20 min at 120°C |

2. Talaromyces medium 2, 3 and 4:

A3 50%, A2 25% and A3 20% are referred to as Talaromyces medium 2 (T2), Talaromyces medium 3 (T3) and Talaromyces medium 4 (T4) respectively. Recipes are given in Table 1.

**Table 1:** Composition of growth media for testing cellulase production in shake flasks and MTP. Sterilization, 20 min at 120°C.

| | Ingredients (g/liter) | | | | |
|---|---|---|---|---|---|
| | A2 media | | A3 media | | |
| Media | T3 | | T4 | T2 | |
| Media composition | 25% | 50% | 20% | 50% | 100% |
| Salt fraction | 30 | 15 | 30 | 15 | 30 |
| Cellulose | 20 | 20 | 30 | 30 | 30 |
| Bacto peptone | 3.75 | 7.5 | 3 | 7.5 | 15 |
| Grain flour | 7.5 | 15 | 6 | 15 | 30 |
| $KH_2PO_4$ | 10 | 10 | 10 | 10 | 10 |
| $CaCl_2.2H_2O$ | 0.5 | 0.5 | 0.5 | 0.5 | 1 |
| Clerol FBA3107 (AF) | 0.4 ml | 0.4 ml | 0.4 ml | 0.4 ml | 0.4 ml |
| pH | 5 | 5 | 5 | 5 | 5 |

*Analogues/Intermediates*

2-dexoxyglucose (Sigma, Cat. No. D6134): stock solution was prepared in water at concentration of 10%.
1-thioglucose (Sigma, Cat. No. T6375): stock solution was prepared in water at concentration of 10%.

*Shake flask growth protocol*

Precultures:

Strains were grown from stocks on Talaromyces agar medium slants for 5-7 days at 40°C; strain stocks are stored at -80°C in 10% glycerol. The grown agar slants need to be stored at room temperature with maximum storage period of two weeks, after this fresh agar slant cultures should be prepared for strain testing.
Spores and mycelium from the agar slants were used to inoculate precultures containing Talaromyes medium 1 (20 ml culture medium in 100 ml Erlenmeyer flask). This was done by pipetting 2 ml of Talaromyces medium 1 onto the surface of the agar slant and repeated pipetting and scraping until the mycelium is removed; the suspended biomass was then dispensed into the inoculation medium. A few drops of the remaining cell suspension were used to inoculate fresh agar slants to secure conservation of the strain. The preculture was then grown for maximum 20 hours at 44°C with shaking (280 rpm).

Production culture:

One or 0.5 ml of preculture was used to inoculate 20 ml of production medium (**Table 1**) in 100 ml Erlenmeyer flask. Cultures were incubated for 4 days at 44°C with shaking (280 rpm).

***Cellulase assays***

1. Wheat Straw assay (WSU assay).

Sample preparation

For shake flask cultures, 10 ml of culture broth was transferred to a 12 ml disposable tube and centrifuged to remove the biomass. One ml of supernatant was then transferred to 96 deepwell plate for storage at -20°C

For 24-well MTP, plates were centrifuged to remove the biomass. One ml of supernatant was then transferred to 96 deepwell plates for storage at -20°C.

For 96-well half deepwell plates, first 300-400 $\mu$l of water in each well was added. Thereafter plates were centrifuged to remove the biomass. A maximum of 200 $\mu$l supernatant was then transferred to 96 deepwell plates for storage at -20°C.

Preparation of pre-treated washed wheat straw substrate.

Dilute-acid pre-treated wheat straw which was washed with water until the solution with wheat straw was pH 6.5 or higher and the mass was homogenised using an ultra-turrax, lyophilized and grinded prior to analysis. To obtain pre-treated wheat straw a dilute acid pre-treatment as described in Linde, M. et al, Biomass and Bioenergy 32 (2008), 326-332 and equipment as described in Schell, D.J., Applied Biochemistry and Biotechnology (2003), vol. 105-108, pp 69-85, may be used.

With 1 WSU is meant 0.119 mg/ml glucose released from 2.1 w/v % washed pre-treated wheat straw by 200 $\mu$l of enzyme mix in 20 hours at 65□C at pH 4.50.

The glucose release is not a linear function of the quantity of enzyme in the composition. In other words, twice the amount of enzyme does not automatically result in twice the amount of glucose in equal time. Therefore, it is preferred to choose the dilution of the composition to be tested for WSU activity such that a WSU does not exceed 40.

Measurement of cellulase activity in WSU

The stored samples were thawed and a two-fold dilution series was made in demineralised water, up to 32 fold. The diluted sample was used to perform two measurements in which 200 $\mu$l of diluted sample was analysed. In the first measurement, 200 $\mu$l diluted sample was transferred to a vial containing 700 $\mu$L water containing 3% ($^w/_v$) dry matter of the pretreated washed wheat straw substrate and 100 $\mu$l of 250 mM citrate buffer, the final pH was adjusted to pH 4.5. In the second measurement, the blank sample, 200 $\mu$l of diluted sample was transferred to a vial that contained 700 $\mu$l of water instead of pretreated washed wheat straw substrate, and 100 $\mu$l of 250 mM citrate buffer, the final pH was adjusted to pH 4.5. The assay samples were incubated for 20 and /or 60 hr at 65 °C. After incubation of the assay samples, 100 $\mu$l of internal standard solution (20 g/L maleic acid, 40 g/L EDTA in $D_2O$) was added. The amount of glucose released, was based on the signal at 5.20 ppm, relative to Dimethyl-sila-pentane-sulfonate determined by means of 1D $^1$H NMR operating at a proton frequency of 500 MHz, using a pulse program with water suppression, at a temperature of 27°C. The WSU number was calculated from the data by subtracting the amount of glucose that was detected in the blank sample from the amount of glucose that was measured in the sample incubated with wheat straw. Wheat straw units are expressed as a function of the dilution factor of the sample.

2. Filter Paper Assay (FPU$_{2\%}$ assay)

Cellulase activity was also measured in term of "filter paper units" (FPU) per milliliter in a Filter Paper Unit assay (FPU assay) of original (undiluted) enzyme solution. The method was modified from the analytical procedure of Adney and Baker (1996, MoA Laboratory Analytical Procedures-006 entitled: Measurement of cellulase activities by Adney and Baker (1996) www.nrel.gov/biomass/analytical procedures.html) to be more sensitive than the standard method that is based on International Union of Pure and applied chemistry (IUPAC) guidelines. For quantitative results the enzyme compositions were compared on the basis of significant and equal conversions. The value of 1.0 mg of reducing sugar as glucose from 50 mg of filter paper (2% conversion instead of the international standard of 4%) in 60 minutes was designated as the intercept for calculation filter paper units (FPU) by IUCA. In this procedure, reducing sugar yield is not a linear function of the quantity of enzyme in the assay mixture, twice the amount of enzyme will not be expected to yield twice the amount of reducing sugar in equal time. The assay procedure therefore involved finding a dilution of the original enzyme stock such that a 0.5 ml aliquot of the dilution will catalyze 2% conversion in 60 minutes, and then calculating the activity (in $FPU_{2\%}$/ml) of the original stock from the dilution required.

FPU was calculated using the following formula:

$$\text{Filter paper activity} = 0.37 / (\text{[Enzyme] releasing 1.0 mg glucose) [Units/ml]}$$

[Enzyme] represents the proportion of the original enzyme solution present in the directly tested enzyme dilution.

### Individual cellulase enzyme assays

#### β-glucosidase (WBDG)

Enzymatic β-glucosidase activity was determined at 37°C and pH 4.40 using para-nitrophenyl-β-D-glucopyranoside (Sigma, Cat.. No. N-7006) as substrate. Enzymatic hydrolysis of pNP-β-D-glucopyranoside resulted in release of para-nitrophenol (pNP) and D-glucose. Quantitatively released para-nitrophenol, determined under alkaline conditions, was a measure for enzymatic activity. After 20 minutes incubation the reaction was stopped by adding sodium carbonate and the absorbance is determined at a wavelength of 405 nm. Beta-glucosidase activity was calculated making use of the molar extinction coefficient of para-nitrophenol (Sigma, Cat. No. N-7660).

The methodology used was an absolute method and the activity is expressed in n(ano)mol released p-nitrophenol per second per ml or per gram sample. The β-glucosidase activity is expressed in White Biotech Beta-D-Glucosidase (WBDG) per gram or per milliliter. One WBDG unit is defined as the amount of enzyme that liberates one n(ano)mol para-nitrophenol per second from para-nitrophenyl-beta-D-glucopyranoside under the defined assay conditions (4.7 mM pNPBDG, pH = 4.40 and T = 37°C). The absolute activity was calculated using standard calculation protocols known in the art.

#### Endoglucanases activity (WBCU)

Endoglucanase catalyses the hydrolysis of carboxymethyl cellulose. The amount of reducing sugars formed during the enzyme reaction was determined with dinitrosalisylic acid reagent. The samples were incubated in the presence of carboxymethyl cellulose (Novacel, ref.394) solution 18 g/L in acetate buffer pH 4.60 at 37°C. The incubation was stopped after 60 minutes by adding sodium hydroxide solution. Samples were boiled for 5 minutes in the presence of dinitrosalisylic acid (Acros 15644500) reagent. After diluting with water, intensity of the colour was measured at 540 nm. The methodology was used as a relative method. The results were related to a cellulase composition with an officially assigned activity. The activity was expressed in WBCU units. WBCU unit is defined as the amount of cellulase that hydrolyses in one hour a number of glycosidic bonds equivalent to the production of 0.5 mg glucose under the condition of the assay. The activity was calculated using standard calculation protocols known in the art, by plotting the deltaOD$_{540}$ versus the activity of samples with known activity, followed by the calculation of the activity of the unknown samples using the equation generated form the calibration line.

### Protein measurement assays

#### 1. Total protein

#### TCA Biuret

The method was a combination of precipitation of protein using trichloro acetic acid (TCA) to remove disturbing substances and allow determination of the protein concentration with the colorimetric Biuret reaction. In the Biuret reaction, a copper (II) ion is reduced to copper (I), which forms a complex with the nitrogens and carbons of the peptide bonds in an alkaline solution. A violet color indicates the presence of proteins. The intensity of the color, and hence the absorption at 546 nm, is directly proportional to the protein concentration, according to the Beer-Lambert law. The standardisation was performed using BSA (Bovine Serum Albumine) and the protein content was expressed in g protein as BSA equivalent/L or mg protein as BSA equivalent /ml. The protein content was calculated using standard calculation protocols known in the art, by plotting the $OD_{546}$ versus the concentration of samples with known concentration, followed by the calculation of the concentration of the unknown samples using the equation generated from the calibration line.

## 2. Individual proteins using PAGE, densitometer and LC/MS/MS measurements

### Sample pre-treatment SDS-PAGE

Based on the estimated protein concentration of the samples the following samples preparation was performed. To 10 $\mu$l sample 40 $\mu$l MilliQ water and 50 $\mu$l TCA (20%) was added to dilute the sample five times (~ 1 mg/ml) and precipitate the proteins. After 1 hour on ice the sample was centrifuged (10 minutes, 14000 rpm). The pellet was washed with 500 $\mu$l Aceton and centrifuged (10 minutes, 14000 rpm). The pellet was treated as described below.

### SDS-PAGE

The pellet was dissolved in 65 $\mu$l of the MilliQ water, 25 $\mu$l NuPAGE™ LDS sample buffer (4x) Invitrogen and 10 $\mu$l NuPAGE™ Sample Reducing agent (10x) Invitrogen. Prior to the the deanuarion step the sample was diluted 5 timnes using a mix of MilliQ; NuPAGE™ LDS sample buffer and 10 $\mu$l NuPAGE™ Sample Reducing in the ratio of 65:25:10. After mixing, the samples were incubated in a thermo mixer for 10 minutes at 70°C. The sample solutions were applied on a 4-12% Bis-Tris gel (NuPAGE™ BisTris, Invitrogen). A sample (10$\mu$l) of marker M12 (Invitrogen) was also applied on the gel. The gel was run at 200 V for 50 minutes, using the XCELL Surelock, with 600 ml 20 x diluted SDS buffer in the outer buffer chamber and 200 ml 20 x diluted SDS buffer, containing 0.5 ml of antioxidant (NuPAGE™ Invitrogen) in the inner buffer chamber. After running, the gel was rinsed twice with demineralised water the gels were fixed with 50% methanol/7% acetic acid solution for one hour and stained with Sypro Ruby (50 ml per gel) overnight. An image was made using the Typhoon 9200 (610 BP 30, Green (532 nm), PMT 600V, 100 micron) after washing the gel with MilliQ water.

### Quantitative analysis of the protein

Using the Typhoon scanner the ratio between protein bands within a lane was determined using standard methods known in the art. The sample was applied in triplicate and the gray values were determined using the program Image quant. Values are expressed as relative % protein to the total protein, calculated using the gray value of the selected protein band relative to the total gray value all the protein bands.

The selected bands were cut with Exquest Spotcutter from BioRad (FE003) and flushed with milliQ water in a 96 wells microtiterplate. The milliQ water was discarded and all bands were cut for a 2nd time to collect more sample material.

### In-gel digestion of proteins for peptides sequence analysis using LC/MS/MS

The gel pieces collected in a microtiterplate were washed with 50 mM $NH_4HCO_3$ pH=7.8. This was done several times, each time shrinking the gel pieces in between the washing steps with acetonitrile to remove any impurities and ensure ideal circumstances for in-gel digestion. The amounts of 50 mM $NH_4HCO_3$ and acetonitrile added vary with the size of the gel pieces; the gel piece should be well emerged in the solutions. Freshly prepared trypsin solution was added and the MTP plate was incubate for at least half an hour on ice or at 4 °C in the refrigerator, followed by overnight incubation at 37 ° with a heated lid on the MTP. After the overnight incubation the gel pieces were sonicated for 1-5 minutes and the fluid fraction transfer into a fresh LoBind MTP. In order to remove as much peptides as possible from the gel pieces as small volume of acetonitrile and formic acid was added to the gel pieces followed by sonification for 1-5 minutes. The fluid fraction was transferred into the LoBind MTP plate and a small volume of formic acid was added.

The amino acid sequence of the tryptic peptides was determined using standard LC/MS/MS analysis as known in the art.

For analyzing the peptide mixtures, a so-called survey scan was used. This method, in which each scan consists of two segments, was defined as follows:

1. full MS scan analysis, for selecting precursor ions for MS/MS based on defined criteria
2. MS/MS of the selected ions to obtain amino acid sequence information.

With this procedure one precursor ion at a time was selected for MS/MS within certain thresholds, which results in MS/MS datasets of peptides, all presenting the amino acid sequence of a part of a protein. All MS/MS spectra were used for protein identification by database searching using the Mascot search engine in the appropriate databases.

3. Individual proteins using APEX proteomics analysis

Materials

LC-MS/MS system consisted of an Accela and an LTQ-Velos from Thermo Fisher (San Jose, CA, USA). Columns were purchased from Agilent : Zorbax 2.1 mm x 5 mm $C_{18}$ column 1.8 □m particles. A Biofuse fresco from Heraeus centrifuge was used for centrifugation of eppendorf tubes, a Beckman Coulter Allegra X-15R was used for centrifugation of Greiner tubes. A thermomixer comfort from Eppendorf (Hamburg, Germany) was used for incubations and Eppendorf Lo-bind tubes were used for all experiments. Database searches were performed, using the Sorcerer 2 (SageN, San Diego, CA, USA) search engine operating the Trans-Proteomics Pipeline (TPP).
The identification results were processed using Absolute Protein Expression software (APEX) http://pfgrc.jcvi.org/index.php/bioinformatics/apex.html, freeware to obtain protein quantities.
Buffers A and B LC-MS grade 0.1% Formic Acid (FA) in water and 0.1% FA in acetonitrile respectively were purchased from Biosolve (Valkenswaard, The Netherlands). Bovine Serum Albumin (BSA) Urea, Iodo Acetamide (IAA) and Trichloroacetic acid (TCA), 6.1 N solution, were purchased from Sigma Aldrich (St. Louis, MO, USA). The TCA solution was 4.5 times diluted to obtain a 20% TCA solution. Dithiothreitol (DTT) was purchased from Roche Applied Science (Indianapolis IN, USA). Formic acid (FA) was purchased from JT Baker (Phillipsburg, NJ, USA). Sequencing grade trypsin was purchased from Roche applied science (Penzberg, Germany).

TCA precipitation and digestion

Samples were carefully de-frozen and stored on ice as much as possible during sample preparation. The samples were diluted to a protein concentration of 5 mg/ml. Hundred μl sample and 50 μl 0.1 mg/ml BSA were 1:1 diluted with 20% TCA. Samples were incubated at 4 °C for 30 minutes and the proteins were pelleted by centrifugation 10 minutes at 13000 rpm 4 °C. The supernatant was removed and the pellets were washed with 200 μl acetone -20 °C. Again the proteins were pelleted by centrifugation 10 minutes at 13000 rpm 4 °C and the supernatant was removed.
The washed pellets were dissolved in 75 μl 8M urea. This solution was diluted with 392.5 μl 100 mM $NH_4HCO_3$. Five μl 500 mM DTT was added and the samples were incubated at room temperature for 30 minutes under maximum agitation in a Thermomixer. The cysteines were alkylated by adding 13.5 μl 550 mM IAA and incubation at room temperature for 30 minutes under maximum agitation in a Thermomixer in the dark. Digestion was performed by adding 20 μl 250 μg/ml trypsin pH 3 and incubation at 37 °C over night under maximum agitation in the Thermomixer. Another 5 μl of 250 μg/ml trypsin pH 3 was added and digestion was continued for 3 hours at 37 °C to ensure completion.
The samples were analyzed on the Accela LTQ-Velos system (Thermo Electron).

- Column: Agilent 2.1 mm x 5 mm $C_{18}$ column 1.8 □m particles
- Gradient 80 minutes from 5-40% AcN 0.1 % Formic Acid
- 2 minutes 40-60% AcN 0.1% Formic Acid
- Flow 400 □L/min
- Injection volume: 20 □l
- Total runtime including injection, column washing and re-equilibration 85 minutes
- MS method: 10th order double play enhanced MS m/z 300-2000 and MS/MS on the top 10 peaks
- Charge state rejection only allowing 2+ and 3+ ions
- Dynamic exclusion: repeat 1, exclusion duration 10 seconds

Data analysis using Sorcerer and Spotfire

The data were searched against the *Talaromyces emersonii* database (TEMER), which was manually edited to contain the sequences of BSA internal standard. Database searching was performed on the Sorcerer 2, using the TPP. Statistical analysis of the data was performed using standard statistical tools.

*Protease assays*

The method applied is used to the determination of protease activity under acid conditions (pH 3.5). The sample is incubated in the presence of haemoglobin (Sigma, Cat. No. H2625) substrate at 60 °C. The reaction is terminated after 60 minutes by adding trichloroacetic acid. The precipitate that is formed is removed by the means of filtration using a Whatman 42 filter. The optical density of the filtrate measured at 280 nm, is compared with those of a calibration line solutions, determined in the same series. The activity is expressed in APU (Acid Protease Unit) per ml or per gram. 1 APU is the amount of enzyme that hydrolyses an amount of substrate per minute, under the condition of the test giving a solution with an optical density at 280 nm equal to the optical density of a solution containing 1.911 $\mu$g tyrosine per ml.

The activity is calculated using standard calculation protocols known in the art, by plotting the Delta $OD_{280}$ versus the activity of samples with known activity, followed by the calculation of the activity of the unknown samples using the equation generated from the calibration line.

***Cellulase plate assay***

Fluorescence cellulase plate assay

Fluorescence compounds 4-methylumbelliferyl-$\beta$-D-cellobioside (Sigma, Cat. No. M6018) and 4-methylumbelliferyl-$\beta$-D-glucose (Sigma, Cat. No. M3633) were used to visualize expression of CBH and $\beta$-glucosidase activity, respectively, of colonies grown on C agar plates. Substrate 4-methylumbelliferyl $\beta$-D-cellobiose (MUC) and 4- methylumbelliferyl $\beta$-D-glucose (MUG) was stored as 10 mM stock solutions in DMSO and immediately before using stocks are diluted 10x with 50 mM sodium acetate buffer, pH 4.5. The solutions obtained were filter sterilized (0.2u) and added to molten agar medium (CTXG) to a final concentration of 0.1 mM and poured into Petri dishes. Different concentrations of glucose were used: none, 0.1%, 0.5%, and 1%. As a control, agar plates were used with 0.5% maltose.

Q trays containing CTXG + fluorescent substrate were inoculated with $10^4$ mutagenized spores from selected mutant libraries. As controls, agar plates were similarly inoculated with reference strains, TEC-147, TEC-123 and TEC-101. During incubation at 40°C, colonies were screened for fluorescence in the presence or absence of glucose by daily examination of Q trays under UV light (366 nm). In total $10^6$ mutated spores were screened on fluorescence agar medium. Mutants that gave strongest fluorescence in presence or absence of glucose were saved for further study.

AZCL xylan (xylanase plate assay

The color compounds AZCL xylan from oat (Megazyme, Cat.no I -AZXOT) was used to visualize expression of xylanase activity of colonies grown on agar plates.

YNB agar was used containing 1% cellulose and AZCL xylan was added to the agar with a final concentration of 0.1 %. Also 2% glucose was added to the agar as needed. Strains to be tested were inoculated (i.e. streaked for single colonies) on these agar plates using spore suspensions from glycerol stocks or well sporulated agar slants. Agar plates were incubated for 2-3 days at 40°C. Quality of growth and size of blue colored halo surrounding the colonies were used to estimate the level of xylanase production among different strains.

***Mutagenesis conditions***

Preparation of spores

Spores are harvested from agar plates with Talaromyces 2 medium agar that were inoculated with a 10 fold diluted spore suspension from the spore stock of strain TEC-101. After incubation for 7 days at 40°C harvesting was done by scraping sporulated mycelium from the agar plates using 10% glycerol solution. Spores were suspended in 10% glycerol and stored at -80° C.

NTG

A *T. emersonii* spore batch ($\geq 10^8$ spores) was inoculated in 20 ml Talaromyes medium 1and this culture is incubated for 4 hr at 45°C with shaking (280 rpm) to start germination of spores. The spore suspension was centrifuged and concentrated by resuspending the pellet in 10 ml 0.01 M TRIS maleic acid buffer pH 8, containing 0.01% TritonX100. The germinated spores were treated with NTG mutagenesis using standard methods known in the art (Cerdá Olmedo, 1987, Mutagenesis. In Phycomyces,(E. Cerdá-Olmedo and E. D. Lipson, eds.), pp. 341-344. CSH Laboratory Press, Cold Spring Harbor, NY, USA).

NTG concentrations used were: 0, 0.05, 0.1 and 0.2 mg/ml. Addition of 10% $Na_2S_2O_3$ solution was used to stop/ inactivate the NTG reaction.

After mutagenesis, the treated cells were washed one time in physiological salts buffer and dilutions ($10^{-1}$-$10^{-5}$/ml) plated onto Talaromyces agar medium plates to determine the cell survival. Plates were incubated at 45°C for 3-7 days for determination of CFU titers.

Each mutant batch (0.5 ml) was used to inoculate one Q-tray (24 cm x 24 cm; VWR) containing TEC agar. The remainder of the mutated cells suspension was stored at -80°C in freeze tubes with 10% glycerol.

The frequency of FoA[r] mutants in the different mutant batches was then determined to assess the quality of the mutant batches. ~10[7] spores per mutant batch were plated onto FOA agar medium. Plates are incubated at 40°C for 3-7 days for determination of CFU titers.

Mutant batches with cell survival rates of 5-25% and that have at least a 10 fold increase in FoA[r] colonies were routinely used for mutant screening.

UV

The germinated spores are treated with UV irradiation using standard methods known in the (Bos, C.J. and Stadler, D, 1996. Mutation. In Fungal Genetics, Principles and Practice (C. J. Bos, ed.), pp 28-31, Marcel Dekker, New York; Bos et al., 1992, Curr. Genet. 21:117-120).

NQO

The germinated spores were treated with NQO using standard methods known in the art (Jerzy Bal et al., 1977, Mutation Research 56:153-156). Briefly, the protocol that is used is comparable to one used for NTG mutagenesis of *Talaromyces* germinating spores._NQO concentrations used were 0.5, 1 and 2.5 $\mu$g/ml per spore suspension. Incubation time in the presence of NQO was 1 hr. Addition of 10% $Na_2S_2O_3$ solution was used to stop/ inactivate the NQO reaction.

***Protoplast fusion to combine mutations***

Protoplast formation

Conditions for protoplast formation were derived from protocols known in the art (Peberdy, 1975, Arch Micro 105:201-205; Peberdy, 1985, J. Gen. Microbiol. 131:2813-2823. *Talaromyces* strains with different selection markers, e.g., FOA (5-fluoro-orotic acid) resistance and DOG (2-deoxy-D-glucose) resistance were inoculated from glycerol stock in Talaromyces medium 1 and grown in shake flasks for 48 hr at 45°C, 280 rpm. Two ml of these inoculation cultures were used to inoculate 500 ml shake flasks with 50 ml of YGG medium (per liter: 8 g KCl, 16 g glucose.H2O, 20 ml of 10% yeast extract, 10 ml of 100x pen/strep, 6.66 g YNB+amino acids, 1.5 g citric acid, and 6 g K2HPO4). Incubation was continued for 16 hours at 45 °C in a rotary shaker at 250-280 rpm. Mycelia from these cultures were than harvested by centrifugation in 50 ml Greiner tubes, for 10 min at 4000 rpm or using Miracloth filter paper (Calbiochem). Pellets were washed and resuspended in 0.6M KCl / 20mM citrate buffer, pH 5.8.

Protoplast isolation

For protoplast formation, per 2 g of mycelium 10 ml of STC buffer (per liter: 218 g Sorbitol (1,2M), 7.35 g CaCl2.2H2O, 10 mM Tris/HCl pH7.5) and 2 ml of glucanex solution (250 mg/ml Glucanex 200G (Novozymes) in $H_2O$) was added. The mixture was incubated in a rotary shaker at 100 rpm at 34°C for 90-150 minutes. Protoplasts were separated from mycelium using a miraclot filter and STC was added to a final volume of 45 ml. The protoplast suspension was centrifuged for 10 minutes at 390 g at 4 °C and the protoplasts were subsequently resuspended in 45 ml of STC. The protoplasts were centrifuged for 10 minutes at 390 g at 4 °C and resuspended in STC-buffer at a concentration of 10[7] - 10[8] protoplasts/ml. The quality and number of protoplasts was verified under a microscope.

Protoplast fusion:

Fusion of protoplasts was induced by mixing of 1 ml of protoplast suspensions with 6 ml of 60% PEG 4000 (Merck), containing 50mM $CaCl_2$, followed by incubation for 15 minutes at room temp. After incubation, PEG is diluted by addition of 30 ml of KCl /citrate buffer and centrifugation for 5 minutes at 2000 rpm.

Pellet is resuspended in 2 ml fresh KCl /citrate buffer and this protoplast suspension was plated on regeneration agar plates. For regeneration Talaromyces agar medium plates were used as described but with addition of sorbitol as osmotic stabilizer, end concentration is 0.8 M. After incubation for 7 days at 40°C spores were harvested from the regeneration agar plates and these spore suspensions were plated on selective agar media to select for fusants with both selectable markers.

*Batch fermentation*

Inoculation procedure:

The content of one vial was added to a pre-culture medium: baffled 2 L-shake flask [20 g/L yeast extract, 20 g/L glucose, pH 6.8 (with KOH), 300 mL medium, steam-sterilized 20 min at 121°C]. Pre-culture was grown for 24-48 h at 48°C and 200 rpm. Timing can be adapted to the shake flask configuration and the vial viability.

Main fermentation procedure:

The medium was composed of a mix of grain flour (3%), cellulose (6%), a nitrogen source (2.5%; examples of nitrogen sources known in the art include soy bean meal, yeast extract, corn steep liquor, ammonia, ammonium salts, nitrate salts.), as well as a salt fraction. The salt fraction was fitting with WO98/37179, Table 1, p. 12. Deviations from this table were: CaCl2.2aq 1.0 g/L, KCI 1.8 g/L citric acid.1aq 0.45 g/L (chelating agent). The medium was steam-sterilized in one fraction. Bioreactors were inoculated at ~10% inoculum ratio and the working volume was 10 L. Process pH was controlled between 5.0-3.0 (using phosphoric acid and ammonia), while temperature at 42-52°C. Airflow was maintained between 0.5 and 1.5 vvm (volume air per volume broth per minute) and the DOT above 30% of the oxygen saturation before inoculation with the aim of agitation. Clerol was used as antifoam on a periodic manner: 2 sec. every 30 min. for the first 24h, then 2 sec. every hour later on.

At the end of the pre-culture phase samples were taken for contamination check, glucose determination and pH measurement. During the main fermentation, samples were taken every 24 h and the following analysis were performed: contamination control, pH measurement, SDS-PAGE gels, total protein concentration determination (TCA Biuret method), Filter Paper Unit activity ($FPU_{2\%}$/ml), β-glucosidase (WBDG) activity, endoglucanases activity (WBCU) and wheat straw assay, as described above.

## EXAMPLE 1

This example describes isolation and characterization of colony morphology variants of *T. emersonii.*

*Talaromyces emersonii* is a thermophilic filamentous fungus. Sporulation on the standard PDA agar medium is very poor ($<10^6$/ml), growth on Talaromyes medium 2 gives improved sporulation ($\sim10^7$/ml). After plating serial dilutions of TEC-1A on Talaromyces agar medium plates, colonies with three different phenotypes can be discriminated (**Figure 1**): TEC-101, distinct white aerial mycelia (80% of population); TEC-102 and TEC-103, reduced aerial mycelia (20% of population; two isolates recovered); TEC-104, no distinct aerial mycelia, brown color (1% of population). The phenotype of TEC-104 remained stable for many generations after growing on agar plates or liquid growth media. However for TEC-101, colony variants similar to TEC-104 continued to be produced at low frequency (<1%).

Sporulation tests indicated that TEC-104 produced the highest spore titers ($>10^7$/ml). However in time-course shake flask experiments, only TEC-101 produced cellulase at levels similar or higher levels compared to the parent TEC-1A (**Figure 2**). Moreover, in 10-liter fermentor batch fermentation both TEC-101 and TEC-1 A produced 5 $FPU_{2\%}$/ml after 96 hrs fermentation, whereas TEC-104 produced less cellulase (2.3 $FPU_{2\%}$/ml). Therefore, TEC-101 gave the best overall characteristics and was chosen for strain improvement.

TEC-101 (also designated as FBG 101) was deposited at CENTRAAL BUREAU VOOR SCHIMMELCULTURES, Uppsalalaan 8, P.O. Box 85167, NL-3508 AD Utrecht, The Netherlands on 30th June 2010 having the Accession Number CBS 127450. Where-ever herein reference is made to "wild-type *Talaromyces* strain" or "wild-type strain" or "wild-type", TEC-101 is meant and/or used.

## EXAMPLE 2

This example describes the isolation of improved cellulase production mutants of *T. emersonii* by high throughput screening of random mutants.

NTG-treated TEC-101 spores were plated from frozen stocks onto Talaromyces agar medium and grown for 4 days at 40°C. Approximately 6000 mutants were transferred into 96 wells microtiter plates (MTP) containing Talaromyces agar medium (200 μl/well). These MTPs, called "masterplates" were incubated at 40°C for 5 days. Several wells were left blank to later contain the TEC-101 non-mutagenized parent and Filtrase® NL enzyme.

The masterplates were replica plated using an automated inoculation device into 24-well MTPs containing Talaromcyes medium 4 (3 ml/well). To do this, physiological salts (150 μl/well) was added to the masterplate wells and the spore suspensions was transferred to the 24-well MTP. In addition, a new 96 well masterplate (200 μl/well Talaromyces agar medium) is inoculated in this protocol. The 24 well plates were incubated in shakers at 46°C and 80% humidity for 96 hours. The cultures were spun down to remove the biomass and one ml supernatant samples were transferred to multiple vials to test for cellulase activity using the wheat straw assay.

Mutants for which the cellulase activity in the growth medium was 20 - 30% higher than the control supernatant from TEC-101 were selected for further screening rounds. These selection rounds were more stringent than the first one, so that it allowed to get rid of false positives.

From each selected mutant from primary screen, two agar slants of each strain were prepared for the secondary screen in shake flask cultures. Spores from these cultures were used to inoculate duplicate shake flask cultures containing Talaromyces medium 2. In some series, a second agar slant was used to first inoculate Talaromyes medium 1 (in shake flask); after growth for 24 hours, 1 ml from this culture was inoculated into the shake flask production medium.

Cellulase activity (wheat straw assay at pH 4.5, 20 hours at 65°C, undiluted samples) of all mutants tested in shake flasks is shown in **Figure 3.** Average of cellulase activities of the TEC-101 control parent strain was 62.6 $\pm$ 4.9 WSU. One particular mutant (DoE 600) had a significant improvement of 63%.

In the tertiary screen, thirty of the best mutant strains from the secondary tests were tested again in shake flask. The procedure was similar to the method used in secondary screen. Strains were singly tested by inoculating spores from agar slant directly into production medium. In **Figure 4** an overview of production of all retested strains is shown.

The first shake flask set (DoE523, 529, 531, 541, 552, 553. 558, 618, and a spontaneous mutant of TEC-101* that produced higher than normal cellulase levels were retested using undiluted supernatant for wheat straw assay; all other were tested using diluted samples to prevent glucose product inhibition of the cellulase. In general, most strains have increased cellulase activity compared to the parent strain. Interestingly, strain DoE 600 showed the highest improvement of more than 100%.

Master cell banks of all 30 selected mutants of TEC-101 from tertiary screen were prepared. Strain ID numbers are TEC-107 - 122, TEC-127 - 130 and TEC-135 - 144.

Dose-response wheat straw assay experiments confirmed that TEC-142 (DoE600) produced almost two-fold more cellulase activity than the control parental strain TEC-101 (**Figure 5**).

## EXAMPLE 3

This example describes the fermentation of cellulase-improved *T. emersonii* mutants.

Three of the cellulase producing mutants from random HTS screening, TEC-121, TEC-137, and TEC-142, and TEC-111; a spontaneous mutant of TEC-101 (previously call TEC-101*) were tested in 10-liter production fermentor as batch fermentations. As controls, additional strains were similarly evaluated: TEC-1A, original starting strain and TEC-101, the common colony variant of TEC-1A, which was the direct parent of the mutants.

At the end of fermentation (96 hr), TCA-Biuret analysis revealed that the total protein concentration was in the range of 9-10 g/kg in all cases. Only for mutant TEC-142 were protein levels significantly higher measuring approx. 16.4 g/kg (**Table 2**).

**Table 2.** Summary of 10-liter fermentor batch fermentation of *T. emersonii* cellulase production mutants and parental strains. nd, not determined.

| Strain | TCA-Biure protein | Cellulase activity after 96 hours fermentation | | BG | EG | Protease (acid) |
|---|---|---|---|---|---|---|
| | (g/kg) | (FPU$_{2\%}$ | (WSU/16xdilution of sample) | (WBDG/ml) | (WBCU/ml) | (APU/ml) |
| | | | | | | |
| TEC-1A | 8.9 | 5.6 | 31.4 | 133 | 3710 | 16200 |
| TEC-101 | 9.1 | 5.1 | 33.1 | 151 | 4580 | nd |
| TEC-111 | nd | 6.4 | 34.9 | 152 | 4310 | nd |
| TEC-121 | 9.7 | 7.2 | 48.0 | 138 | 3790 | nd |
| TEC-137 | 9.5 | 6.0 | 30.8 | 172 | 2990 | nd |
| TEC-142 | 16.4 | 15.8 | 70.2 | 155 | 10200 | 9500 |

FPU analysis results showed that four mutants TEC-111, TEC-121, TEC-137, and TEC-142 produced significantly more cellulase activity (higher than 10%) than the TEC-101 parent strain (measured as FPU$_{2\%}$/ml after 96 h fermentation and taking into account a 10% deviation of the FPU assay). Moreover, TEC-142 produced the highest level, 15.8 FPU$_{2\%}$/ml, which represents a 3-fold improvement over its parent TEC-101 (5.1 FPU$_{2\%}$/ml) (**Table 2**). For TEC-142 mutant, enzyme assays showed at least a two-fold increase in EG levels (10200 WBCU/ml) and wheat straw unit (70.2 WSU/16-fold dilution), but no increase in BG levels (~155 WBDG/ml) compared to the mother strain TEC-101 (4580 WBCU/ml, 33.1 WSU/ml and 151 WBDG/ml, respectively). This is in contrast with mutant TEC-137 in which the increase in FPU level (5.9 FPU$_{2\%}$/ml; 20%) correlated with an increase in BG activity (172 WBDG/ml; 14%), rather than in EG levels (2990 WBCU/ml; 35% decrease) (**Table 2**) The second best mutant was found to be TEC-121 exhibiting 7.2 FPU$_{2\%}$/ml (40% improvement) and 48 WSU/ml (45% improvement), while on the contrary BG and EG activities were lower than the mother strain. Also noted was a two-fold decrease in acid protease activities.

Dose-response wheat straw assay experiments confirmed that TEC-142 produced almost two fold more cellulase activity than the control strain TEC-1A or the parental strain TEC-101 (**Figure 6**). Interestingly, a low, but significant level of released cellobiose was detected by enzyme produced from this mutant.

Finally, SDS-PAGE analysis of fermentations showed cellulase activity is produced at the latter stages of the fermentation (72-96 h), while in the beginning mainly biomass is formed. Additionally, it was made clear that the TEC-137 mutant produced the highest level of secreted proteins after 48 h. This early secretion is due to the fast growing phenotype of these mutants in lab-scale fermentors or in the pre-culture.

## EXAMPLE 4

This example describes the characterization of the cellulase-improved *T. emersonii* mutant TEC-142.

Serial dilutions of TEC-142 and the parental strain TEC-101 were plated on Talaromyces agar medium plates and grown for 96 hr at 46°C. Colonies of TEC-142 appear to be smaller than TEC-101 and the color of the colonies had also changed to a greenish tinge. Growth was also tested on agar plates containing 3% pre-treated, washed wheat straw. After 96 hours at 46°C, no difference in colony size of TEC-142 and TEC-101 was observed. No growth difference was also observed between TEC-142 and TEC-101 when both were plated onto C-agar with and without the addition of maltose (1 %). Also on halo C-agar plates containing pretreated, washed wheat straw (3%), no difference in halo size was observed between the two strains. Finally TEC-142 did not display a higher resistance to glucose analogs, 2-deoxyglucose or 1-thioglucose at MIC levels compared to TEC-101.

SDS PAGE analysis of supernatants of 10-liter fermentor batch fermentations of TEC-142 coupled with densitometer quantification and LC/MS/MS identification verified that the protein level of the cellulase enzymes had increased. In the parent strain TEC-1A at least four specific cellulases were identified: cellobiohydrolase I (CBH I) plus cellobiohydrolase II (CBH II), endoglucase (EG), beta-glucosidase (BG). Also one hemicellulases was identified, xylanase (**Table 3**).

Table 3. Comparison of the relative extracellular protein levels of TEC-1A and TEC-142. Supernatants from 96 hr 10-liter fermentor batch fermentations of TEC-1A and TEC-142 were subjected to SDS-PAGE and extracellular proteins were identified by LC/MS/MS using publicly known databases, and their levels quantified by densitometer measurements. Values are expressed as relative % proteins to the total protein, calculated using the gray value of the selected protein band relative to the total gray value all the protein bands.

| Protein size (kDa) | TEC-1A Relative intensity %* | TEC-142 Relative intensity %* | Identified protein class using in gel digestion followed by LC/MS/MS analysis |
|---|---|---|---|
| ~ 92 | 3 | 1 | BG |
| ~48 - 55 | 47 | 45 | CBH I / CBH II |
| ~ 36 - 44 | 11 | 22 | EG |
| * Values are expressed as relative % proteins to the total protein, calculated using the gray value of the selected protein band relative to the total gray value all the protein bands. | | | |

Comparison of this profile with the extracellular protein profile of TEC-142 shows significant differences. Most important EG protein was increased 2-fold, which corresponded to the increase in EG enzymatic activity. Interestingly no significant change in BG or CBH I+II protein levels was observed.

Finally, as with original *Talaromyces* TEC-1A strain, at least two different colony morphologies of TEC-142 were observed when plated on various agar medium: the most prevalent (90% of the total) is TEC-101-like (colonies with distinct aerial mycelium) and the least prevalent (10% of the total) is TEC-104-like (no distinct aerial mycelia, brown color). These two variants were purified and renamed TEC-146 and TEC-147, respectively. Sporulation tests indicated that TEC-147 produced the highest spore titers (>10[7]/ml). However unlike the previous time-course shake flask experiments of TEC-101 and TEC-104, TEC-147 produced 10% more cellulase activity (in the wheat straw assay using 8-fold dilution of supernatant) than either TEC-148 or TEC-142 in Talaromyes medium 2 after 96 hr growth at 46°C. This was subsequently confirmed in 10-liter Eschweiler batch fermentation. After 96 hour growth, TEC-146 produced 14.9 $FPU_{2\%}$/ml and 14.0 mg/ml protein (TCA-Biuret assay), whereas TEC-147 produced 17.3 $FPU_{2\%}$/ml and 17.4 mg/ml protein. When the fermentation of TEC-147 was extended to 120 hours, 20.5 $FPU_{2\%}$/ml and 19.4 mg/ml protein were detected in the supernatant.

Thus mutant TEC-147 combines both improved cellulase production and good sporulation properties, which facilitates further improvement of the strain by classical or genetic engineering methods.

## EXAMPLE 5

This example describes the analysis of the cellulase-improved *T. emersonii* mutant TEC-142 in Simultaneous Saccharification Fermentation (SSF) application.

Batch SSF experiments were carried out to compare the performance of the Cellulase enzyme produced by mutant TEC-142 with Filtrase® NL enzyme. Filtered fermentation broth prepared in Example 3 was used in these experiments. SSF conditions are known in the art and are described in patent application WO2010018105. Saccharification of pretreated, washed, wheat straw was at 56°C for 20 hours, at a starting pH 5. The enzymes compositions were added directly to the fermentation batches at various dosages determined as mg TCA-Biuret protein per g Glucan+Xylan content. *S. cerevisiae* (baker's yeast) was added at a concentration of 0.5 g/l and fermentation was carried out at 33°C; the fermentation medium was supplemented with 0.025% $KH_2PO_4$ and 0.05% $(NH_4)_2SO_4$. Ethanol production was measured at the end of fermentation (168 hours) using NMR. To do this, approximately 2 ml of mash was taken from the each flask, samples centrifuged at 4000 rpm for 7 minutes and supernatant was transferred to multiple vials. The samples were kept at -18°C freezer until analyzed.

As shown in **Figure 7**, the dose-response curves of ethanol production from pre-treated, washed wheat straw using the same dosages of Filtrase® NL and cellulase mixture from TEC-142 are almost identical. However, since there is twice as much protein produced by this mutant than wild-type strain, 50% less volume is required based on the end of fermentation filtrate, resulting in a significant cost savings.

TEC-142 was deposited at CENTRAAL BUREAU VOOR SCHIMMELCULTURES, Uppsalalaan 8, P.O. Box 85167, NL-3508 AD Utrecht, The Netherlands on 1st July 2009 and has the Accession Number CBS 124902.

## EXAMPLE 6

This example describes the isolation of mutants of *T. emersonii* TEC-147 that are glucose deregulated for cellulase production by rational selection using glucose analogs.

MIC values of various glucose analogs (2-deoxyglucose, 1-thioglucose, 5-thioglucose) are determined by examining growth after inoculation of 0.1 ml dilutions of $10^7$/ml spore suspensions of TEC-142 on YNB+ agar plates with increasing concentrations of analogs.

Results indicate that the MIC levels for these two strains are: 2-deoxyglucose - 0.5 g/liter and 1-thioglucose - 4 g/liter.

Mutant batches of TEC-147 prepared using UV, NTG, and a combination of NTG and NQO are then plated onto agar plates ($10^6$ spores per plate) containing glucose analogues. YNB+ agar plates + 0.1% deoxyglucose or 0.4% 1-thioglucose are used to select for the desired glucose analogue resistant mutants.

For each selection, 100 resistant mutants are picked from agar plates and transferred to Talaromyces agar medium slants. Slants are used for inoculation of two sets of duplicate shake flasks with Talaromyces medium 2 and the cultures were grown at 46°C with shaking (280 rpm). One set is harvested after 48 hour growth when glucose was just exhausted, and the other set was harvested after 96 hours growth when cellulase levels are normally the highest. Comparison of the ratio of cellulase enzyme production at 48 and 96 hours is used to identify mutants that are glucose derepressed with respect to cellulase production. Glucose derepression is verified by growing the mutant in Talaromyces medium 2 containing 5% glucose for 96 hours in shake flasks cultures and detecting enzyme production in the wheat straw assay that is at least 25% of the activity of the unmutated parent strain when similarly grown in Talaromyces medium 2 without glucose.

From this screen, at least one mutant is isolated that gave significantly higher ratio of enzyme production at 48 and 96 hours relative to the parental control and produces cellulase activity that is at least 25% of the activity of the unmutated parent strain when similarly grown in Talaromyces medium 2 without glucose for 96 hours. The invention thus provides a Talaromyces strain being glucose-derepressed with respect to cellulose production.

## EXAMPLE 7

This example describes the isolation of mutants of *T. emersonii* TEC-147 that produce glucose cellulase at earlier growth times using high throughput screening of random mutants.

TEC-147 spores treated with UV, NTG or a combination of NTG and NQO are plated from frozen stocks onto Talaromyces agar medium containing penicillin (6.7 mg/liter) and streptomycin (10 mg/liter) antibiotics, and grown for 4 days at 40°C. A total of 11, 000 mutants are grown in 96 wells microtiter plates (MTP) containing Talaromyces agar medium (200 $\mu$l/well) with antibiotics. These MTPs, called "masterplates" are incubated at 40°C, for 5 days.

The masterplates are replica plated into 96-well half deepwell plates containing Talaromyces medium 3 (300 $\mu$l/well). After inoculation the half deepwell plates are covered with a breathable seal and the lid was replaced. The 96 well plates are incubated in shakers at 44°C and 80% humidity for 96 hours. After addition of 500 $\mu$l/well water, the cultures are spun down to remove the biomass and 50 $\mu$l of supernatant is transferred to multiple vials to test for cellulase activity using the wheat straw assay.

The top 10% of the mutants (1000 mutants) for which the cellulase enzyme activity in the growth medium is significantly higher than the control supernatant from TEC-147 are selected for a second round screening in a 24-well MTP format using Talaromyces medium 4 as described in Example 2. To do this, hits from masterplates are used to prepare new 96 well masterplates (200 $\mu$l/well Talaromyces agar medium). The new masterplates are used to inoculate 24 well plates in duplicate: one set is incubated in shakers at 40°C and 80% humidity for 72 hours when glucose was just exhausted, and the other set was harvested after 144 hours growth when cellulase levels are the highest. Comparison of the ratio of cellulase production at 72 and 144 hours is used to identify mutants that produce cellulase activity earlier than the parental control (TEC-147). The cultures are spun down to removed the biomass and supernatant is transferred to multiple vials to test for cellulase activity using the wheat straw assay

The top 10% of the mutants (100 mutants) for which the cellulase activity in the growth medium is significantly higher than the control supernatant from TEC-147 are selected for a third round screening in a shake-flask cultures. This screening round is more stringent than the first two, so that it allowed to get rid of false positives.

From each selected mutant from primary screen, two agar slants of each strain are prepared for the secondary screen in shake flask cultures. Spores from these cultures are used to inoculate duplicate shake flask cultures containing Talaromyces medium 2 production medium. In some series, a second agar slant is used to first inoculate Talaromyces medium 1 (in shake flask); after growth for 24 hours, 1 ml from this culture is inoculated into the shake flask production medium:

Cellulase activity (wheat straw assay at pH 4.5, 20 hours, diluted samples) of all mutants are tested in shake flasks and master cell banks of the top 10% (10 mutants) are prepared. All ten mutants are evaluated in 10-liter fermentor fermentation using the batch protocol described in Example 3. One mutant is found that produced the highest and earliest level of cellulase activity compared to the TEC-147 parental control.

## EXAMPLE 8

This example describes the combination of beneficial mutations of *T. emerersonii* cellulase improved mutants by protoplast fusion.

A FoA[r] variant of TEC-142, TEC-147 from Example 2, or an improved mutant thereof from Example 7 is crossed with a DG[r] or TG[r] mutant of TEC-142 or TEC-147 from Example 6 that is glucose derepressed for cellulase production. Greater than $10^7$ protoplasts from each strain are mixed together and PEG4000 + 50mM $CaCl_2$ is added to an end concentration of 40% PEG4000. After mixing and incubation for 15 minutes at room temperature, the protoplast mixture was washed with KCl / citrate buffer as described. After washing serial dilutions of the protoplast mixture are plated onto Talaromyces agar medium plus sorbitol as described. After incubation for 7 days at 40 °C, spores are harvested from regeneration agar plates, serial dilutions made, and 0.1 ml is spread onto Talaromyces agar medium plates to obtain single colonies. In some cases, 5-Fluoro orotic acid and deoxyglucose or 5-Fluoro orotic acid and thioglucose are added to the plates to select for recombinant fusants.

Approximately 100 single colony isolates are tested for cellulase production in the 24 well plate screening assay. The best 50 cellulase producing isolates are retested in shake flasks using the TEC medium 2 with or without 5% glucose and using TEC142 or TEC-147 as a reference strain. Five strains are selected with significantly improved cellulase production yet still glucose de-repressed for cellulase production.

Two strains, TEC-PF1 and TEC-PF2, are selected for cellulase production test on 10L scale and showed >20% improvement of cellulase production compared to TEC-142 or TEC-147 and still being glucose derepressed.

## EXAMPLE 9

This example describes the isolation of mutants of *T. emersonii* TEC-142 and TEC-147 that are glucose derepressed for cellulase production by rational selection using 2-deoxyglucose.

The MIC value of 2-deoxyglucose (DOG) was determined by examining growth after inoculation of 0.1 ml dilutions of $10^7$/ml spore suspensions of TEC-142 on YNB+ agar plates with increasing concentrations of analog. Results indicate that the MIC level for this strain was 0.5 g/liter.

Mutant batches of TEC-142 prepared using a combination of NTG and NQO were then plated onto YNB+ agar plates containing 0.1% deoxyglucose ($10^6$ spores per plate). Approximately 300 DG$^r$ mutants are picked from agar plates and transferred to Talaromyces agar medium slants. After sporulation, mutants were purified again on YNB+ agar plates containing 0.1% deoxyglucose. After incubation for 3-4 days at 40°C, single colonies were transferred to Talaromyces agar medium slants. Spores from these slants were used for inoculation of two sets of duplicate shake flasks with Talaromyces medium 2 and the cultures were grown at 46°C with shaking. One set was harvested after 48 hour growth when glucose was just exhausted, and the other set was harvested after 96 hours growth when cellulase levels are normally the highest. Comparison of the ratio of cellulase enzyme production at 48 and 96 hours was used to identify mutants that are glucose derepressed with respect to cellulase production. At least three mutants were identified with a high ratio of TEC production between the two time points (>0.5 ratio), DOG32, DOG33, and DOG264. Glucose derepression was verified by growing the mutants in Talaromyces medium 2 containing 5% glucose over 96 hours in shake flasks cultures and monitoring enzyme production at 24, 48, 72, and 96 hours. Two mutants, DOG33 and DOG264 produced significant quantities of TEC in the presence of glucose compared to the control strains TEC-101, TEC-142 and TEC-147, which did not produce detectable levels of TEC over the same time period **Table 4.**

**Table 4.** Time-course production of TEC production of mutants grown in the presence of 5% glucose and 3% cellulose.

| Strain | TEC production (wsu/8x dilution of sample) | | | |
| --- | --- | --- | --- | --- |
| | 24 hr | 48 hr | 72 hr | 96 hr |
| DOG32 | <1 | <1 | <1 | <1 |
| DOG33 | <1 | 10 | 12 | 14 |
| DOG264 | <1 | 6 | 9 | 12 |
| TEC-101 | <1 | <1 | <1 | <1 |
| TEC-142 | <1 | <1 | <1 | <1 |
| TEC-147 | <1 | <1 | <1 | <1 |

From this screen, DOG33, (renamed TEC-152) was chosen for further study. Comparison of TEC production in SF cultures containing Talaromyces medium 2 with or without 5% glucose showed that glucose derepression was 74% (i.e. e. the mutant produced 25% less TEC in the presence glucose compared to levels produced in the absence of glucose in SF cultures after 96 hr incubation). SDS-PAGE confirmed the production of cellulose-specific protein bands when TEC-152 was grown in the presence of glucose.

When TEC-152 colonies were streaked onto C-medium containing 5% glucose and either of the fluorescence indicators MUG and MUC, an intense <u>fluorescence</u> was detected after 72 hr growth at 46°, whereas no or very little fluorescence was detected with either TEC-142. This indicates that CBH and β-glucosidase activities were significantly increased when cells are grown in the presence of glucose, which is as expected of glucose derepression.

Similarly when TEC-152 was plated onto C medium containing 5% glucose and AZCL xylan, an intense blue color was detected after 72 hr growth at 46°C, whereas no or very little color was detected with either TEC-142. This indicates that xylanase activity was significantly increased when cells are grown in the presence of glucose, which is as expected of glucose derepression.

A similar approach was used to obtain glucose derepressed mutants of TEC-147. Mutant batches of TEC-142 prepared using a combination of NTG and NQO were plated onto YNB+ agar plates containing 0.1% deoxyglucose ($10^6$ spores per plate). Approximately 400 DG$^r$ mutants are picked from agar plates and transferred to Talaromyces agar medium slants. After sporulation, mutants were purified again on YNB+ agar plates containing 0.1% deoxyglucose. After incubation for 3-4 days at 40°C, single colonies were transferred to Talaromyces agar medium slants. Spores from these slants were used for inoculation shake flasks with Talaromyces medium 2 containing 5% glucose and the cultures were grown at 46°C with shaking. Several mutants were obtained that produced significant levels of TEC at 72 hrs (>10 wsu/8x dilution of sample), whereas the TEC-147 parental control did not produce significantly detectable levels of TEC (< 1 wsu/8-fold dilution of sample). Three mutants were saved for further study base on their superior TEC production over 120 hrs growth in SF studies using TEC medium containing 5% glucose: TEC-162 (DOG-540), TEC-164 (DOG-563), and TEC-165 (DOG-568). Of the three, TEC-165 was the best mutant, producing ~15% more TEC than TEC-147 when grown for SF cultures for 96 hours in TEC medium.

When TEC-165 colonies were streaked onto C-medium containing 5% glucose and either of the fluorescence indicators MUG and MUC, an intense fluorescence was detected after 24 hr growth at 46°C, whereas no or very little fluorescence was detected with either TEC-142 or TEC-147. This indicates that CBH and β-glucosidase activities were significantly increased when cells are grown in the presence of glucose, which is as expected of glucose derepression.

Similarly when TEC-165 was plated onto C medium containing 2% glucose and AZCL xylan, an intense blue color was detected after 72 hr growth at 40°C, whereas no or very little color was detected with either TEC-142 and TEC-147. This indicates that xylanase activity was significantly increased when cells are grown in the presence of glucose, which is as expected of glucose derepression.

Comparison of TEC-165 and TEC-152 indicated that TEC-165 was the better glucose derepressed mutant by producing >50% more TEC than TEC-152 in TEC medium containing 5% glucose for 96 hours, despite the fact that TEC-165 was only 50% glucose derepressed (i.e. the mutant produced 50% less TEC in the presence glucose compared to levels produced in the absence of glucose in SF cultures after 96 hr incubation).

SDS-PAGE confirmed the production of cellulose-specific protein bands when TEC-165 was grown in the presence of glucose. However the cellulose-specific protein bands were more intense compared to those from TEC-152.

## EXAMPLE 10

This-example describes the isolation of mutants of *T. emersonii* TEC-147 that produce higher glucose cellulase levels at earlier or later growth times using high throughput screening of random mutants.

TEC-147 spores treated with UV, NTG or a combination of NTG and NQO are plated from frozen stocks onto Talaromyces agar medium containing penicillin (6.7 mg/liter) and streptomycin (10 mg/liter) antibiotics, and grown for 4 days at 40°C. A total of 11, 000 mutants are grown in 96 wells microtiter plates (MTP) containing Talaromyces agar medium (200 $\mu$l/well) with antibiotics. These MTPs, called "masterplates" are incubated at 40°C, for 5 days.

The masterplates were replica plated into 96-well half deepwell plates containing Talaromyces medium 3 (300 $\mu$l/well). After inoculation the half deepwell plates were covered with a breathable seal and the lid was replaced. The 96 well plates were incubated in shakers at 44°C and 80% humidity for 96 hours. After addition of 500 $\mu$l/well water, the cultures are spun down to remove the biomass and 50 $\mu$l of supernatant is transferred to multiple vials to test for cellulase activity using the wheat straw assay.

The top 10% of the mutants (>1100 mutants) for which the cellulase enzyme activity in the growth medium was significantly higher than the control supernatant from TEC-147 were selected for a first round screening in a 24-well MTP format using Talaromyces medium 4 as described in Example 2. To do this, hits from.masterplates were used to prepare new 96 well masterplates (200 $\mu$l/well Talaromyces agar medium). The new masterplates was used to inoculate 24 well plates (with breathable seal and lid) in duplicate: one set was incubated in shakers at 40°C, and 80% humidity for 72 hours when glucose was just exhausted, and the other set was harvested after 144 hours growth when cellulase levels are the highest. Comparison of the cellulase production at 72 and 144 hours to the controls was used to identify mutants that produce cellulase activity earlier or later than the parental control (TEC-147). The cultures were spun down to remove the biomass and supernatants were transferred to multiple vials to test for cellulase activity using the wheat straw assay. In all 24 mutants were saved (Group A) in TEC activity was significantly higher than TEC-147. Another 72 mutants were recovered (Group B) in which the TEC production at 144 hrs was significantly higher than the control supernatant from TEC-147. Of these 96 mutants, 11 were the same (Group C). Both sets of mutants were then tested in shake-flask cultures for increased TEC production.

To do this, agar slants of each strain was prepared for the primary screen. Spores from these cultures were used to inoculate shake flask cultures containing Talaromyces medium 2 medium. The mutants were grown for 96 hours and enzyme production was monitored at 24, 48, 72, and 96 hours. Cellulase activity (wheat straw assay at pH 4.5, 20 hours, 8x diluted samples) indicated that one mutant, EVE750 produced 25-50% more TEC than the parental control, TEC-147 as shown in Table 5. This mutant was from Group B.

Table 5. Time-course production of TEC production of mutants grown in SF culture containing TEC medium for 120 hours.

| Strain | TEC production (wsu/8x dilution of sample) | | |
|---|---|---|---|
| | 72 hr | 96 hr | 120 hr |
| EVE750 | 22 | 37 | 34 |
| TEC-101 | 9 | 11 | 10 |
| TEC-147 | 18 | 24 | 26 |

This mutant was saved for further study and renamed TEC-180. Individual colonies of TEC-180 were prepared by restreaking in TEC agar medium and 10 colonies were retested for TEC production. Only two of the colonies, renamed TEC-192 and TEC-193 produced >25% more TEC than the TEC-147 parental control strain. WCB of TEC-180, TEC-192 and TEC-193 were prepared. Dose-response wheat straw assay experiments confirmed that TEC-192 and TEC-193 produced >25% more cellulase activity than the control parental strain TEC-147.

## EXAMPLE 11

This example describes the fermentation of the glucose derepressed mutant, TEC-165 and further cellulase-improved *T. emersonii* mutants TEC-192 and TEC-193. The afore-mentioned mutants were tested in 10-liter production fermentor as batch fermentations.

Mutant TEC-165 exhibited a long lag-phase of approx. 24-30 h most probably due to a relatively poor growth in the pre-culture. For this reason, growth of this mutant prolonged for one more day and the end of fermentation was after 117 h. On the contrary, for mutants TEC-192 and -193 there was hardly any lag-time observed and the fermentation was ended after 91 h.

At the end of fermentation, TCA-Biuret analysis revealed that the total protein concentration was -17 g/kg for TEC-192 and -193, while for TEC-165 it was bit higher (18.8 g/kg). These values were in the same range as found for the parental strain TEC-147 (17.5 g/kg) (**Table 6**).

**Table 6.** Summary of 10-liter fermentor batch fermentation of *T. emersonii* cellulase production mutants TEC-165, -192, -193 and their parental strain TEC-147. nd, not determined.

| Strain | Time (h) | TCA-Biuret protein | Cellulase activity | | BG |
|---|---|---|---|---|---|
| | | (g/kg) | (FPU$_{2\%}$/ml) | (WSU/16x dilution of sample) | (WBD G/ml) |
| | | | | | |
| TEC-147 | 95 | 17.4 | 17.3 | 49* | 144 |
| TEC-165 | 117 | 18.8 | 18.5 | 41.2 | nd |
| TEC-192 | 91 | 17.1 | 20 | 51.6 | nd |
| TEC-193 | 91 | 16.9 | 20 | 50.3 | nd |
| * different wheat straw batch. | | | | | |

FPU analysis results showed that all three mutants produced more cellulase activity than the TEC-147 parent strain (measured as FPU$_{2\%}$/ml at the end of fermentation). TEC-192 and -193 produced the same cellulase activity after 91 h that was significantly higher (15%) than TEC-147. Taking into account the higher cellulose activity and the shorter total fermentation time compared to the parental strain, the effect on productivity is even greater (21% increase). Based on the wheat straw until assay the difference between mutants TEC-192 and -193 and the parental strain is much lower, since in the former case a new wheat straw batch was used that in general generates 5-10% lower activity values than the old one. For TEC-165 the increase in cellulose activity (measured as FPU$_{2\%}$/ml at the end of fermentation) was 7%, which falls within the error range of 10% that the FPU assay has (**Table 6**). Because of the longer lag-phase that mutant TEC-165 exhibited, productivity was lower compared to the parental strain. WSU activity was also lower, but no concrete conclusions can be draw since a different wheat straw batch was used for the analysis. Finally, SDS-PAGE analysis of all fermentations showed cellulase activity is produced at the latter stages of the fermentation (72-117 h), while in the beginning mainly biomass is formed.

## EXAMPLE 12

This example describes the isolation of mutants of *T. emersonii* TEC-180 and its isolates TEC-192 and TEC-193 that are glucose deregulated for cellulase production by rational selection using 2-deoxyglucose.

MIC values of 2-deoxyglucose was determined by examining growth after inoculation of 0.1 ml dilutions of $10^7$/ml spore suspensions of TEC-180 on YNB+ agar plates with increasing concentrations of analogs. Results indicate that the MIC level for this strain was 0.5 g/liter.

Mutant batches of TEC-180 prepared using a combination of NTG and NQO were then plated onto YNB+ agar plates containing 0.1% deoxyglucose ($10^6$ spores per plate). Approximately 100 DG$^r$ mutants are picked from agar plates and transferred to Talaromyces agar medium slants. After sporulation, mutants were purified again on YNB+ agar plates containing 0.1% deoxyglucose. After incubation for 3-4 days at 40°C, single colonies were transferred to Talaromyces agar medium slants. Spores from these slants were used for inoculation shake flasks with Talaromyces medium 2 containing 5% glucose and the cultures were grown at 44°C with shaking (280 rpm). Several mutants were obtained that produced significant levels of TEC at 72 hrs (>10 wsu/8x dilution of sample), whereas the TEC-180 parental control did not produce significantly detectable levels of TEC (< 1 wsu/8-fold dilution of sample). Three mutants were saved for further study base on their superior TEC production at 72 hrs growth in SF studies using TEC medium containing 5% glucose: TEC-199 (DOG158A), TEC-200 (DOG172A), and TEC-201 (DOG173A). Comparison of these mutants to TEC-165 indicated that all produced higher TEC titers than TEC-165 after 72 hrs growth in TEC medium containing 5% glucose (**Table 7**). SDS-PAGE confirmed the production of cellulose-specific protein bands when each mutant was grown in the presence of glucose.

**Table 7.** Time-course production of TEC production of mutants grown in the presence of 5% glucose and 3% cellulose.

| | TEC production (wsu/8x dilution of sample) | |
|---|---|---|
| Strain | 72 hr | 96 hr |
| TEC-199 | 10 | 22 |
| TEC-200 | 15 | 21 |
| TEC-201 | 10 | 22 |
| TEC-147 | <1 | 5 |
| TEC-180 | <1 | 4 |
| TEC-165 | 12 | 15 |

From this screen, all three mutants were chosen for further study. Comparison of TEC production in SF cultures containing Talaromyces medium 2 with or without 5% glucose showed that glucose derepression for all was 50% (i.e. the mutant produced 50% less TEC in the presence glucose compared to levels produced in the absence of glucose in SF cultures after 96 hr incubation). Moreover, the three mutants all retained their high TEC production level in TEC medium (no glucose) produced 25-50% more TEC than the TEC-147 parent. In addition, plate assays containing the fluorescence indicators MUG or MUC, or the color indicator AZCL xylan, indicated that CBH and β-glucosidase activities, and xylanase activity are glucose derepressed.

A similar approach was used to obtain glucose derepressed mutants of TEC-192 and TEC-193. Mutant batches of TEC-192 and TEC-193 prepared using a combination of NTG and NQO were plated onto YNB+ agar plates containing 0.1% deoxyglucose ($10^6$ spores per plate). Approximately 75 DG$^r$ mutants are picked from agar plates and transferred to Talaromyces agar medium slants. After sporulation, mutants were purified again on YNB+ agar plates containing 0.1% deoxyglucose. After incubation for 3-4 days at 40°C, single colonies were transferred to Talaromyces agar medium slants. Spores from these slants were used for inoculation shake flasks with Talaromyces medium 2 containing 5% glucose and the cultures were grown at 46°C with shaking. Several mutants were obtained that produced significant levels of TEC at 72 hrs (>10 wsu/8x dilution of sample), whereas the TEC-192 and TEC-193 parental controls did not produce significantly detectable levels of TEC (< 1 wsu/8x dilution of sample). Four mutants were saved for further study base on their superior TEC production over 72 hrs growth in SF studies using TEC medium containing 5% glucose: TEC-205 (DOG235A), TEC-207 (DOG340A), TEC-209 (DOG343A), and TEC-210 (DOG344A) (**Table 8**). Comparison of TEC production in SF cultures containing Talaromyces medium 2 with or without 5% glucose showed that glucose derepression for all was 50% (i.e. the mutant produced 50% less TEC in the presence glucose compared to levels produced in the absence of glucose in SF cultures after 96 hr incubation). However mutant TEC-209 (DOG343A), was >60% glucose derepressed based on this measurement, but the TEC level was lower than the other mutant when grown in A3 50% without glucose. Finally, the three mutants all retained their high TEC production level in TEC medium (no glucose) produced 25-50% more TEC than the TEC-147 parent. All four mutants were chosen for further study. In addition, plate assays containing the fluorescence indicators MUG or MUC, or the color indicator AZCL xylan, indicated that CBH and β-glucosidase activities, and xylanase activity are glucose derepressed in these mutants.

**Table 8.** Time-course production of TEC production of mutants grown in the presence of 5% glucose and 3% cellulose.

| | TEC production (wsu/8x dilution of sample) | |
|---|---|---|
| Strain | 48 hr | 96 hr |
| TEC-205 | 14 | 21 |
| TEC-207 | 13 | 10 |

(continued)

| Strain | TEC production (wsu/8x dilution of sample) | |
|---|---|---|
| | 48 hr | 96 hr |
| TEC-209 | 15 | 19 |
| TEC-210 | 12 | 17 |
| TEC-147 | 1 | 6 |
| TEC-192 | 0 | 6 |
| TEC-193 | 0 | 3 |
| TEC-165 | 12 | 9 |

## EXAMPLE 13

This example describes the characterization of glucose derepressed *T. emersonii* mutants.

TEC-165 showed several different properties to its parent TEC-147 in terms of growth and enzyme production. First, when plated on agar medium, colonies of TEC-165 appear were distinctly smaller than the parent strain TEC-147, indicating significant slower growth (**Figure 8**). This slow growth property was also observed in shake flask cultures. Similar slow growth properties were also observed with the other glucose derepressed strains TEC-152, TEC-199, TEC-200, TEC201, TEC-205, TEC-209, and TEC-210.

Enzyme plate assays also indicated a significant difference in glucose derepressed mutants from their parent strains in terms of enzyme production in the presence of glucose. When grown on TEC medium containing 2-5% glucose and the fluorescent substrates 4-methylumbelliferyl-β-D-cellobioside or 4-methylumbelliferyl-β-D-glucose which are used to visualize expression of CBH I+II and β-glucosidase activity, respectively, TEC-165 colonies produced a more intense fluorescence compared to TEC-101 or TEC-147 (**Figure 9**), indicating more CBH and β-glucosidase activity in TEC-165 than in TEC-147. Similar results were observed with all other glucose derepressed mutants.

Finally in addition to showing an increase in lignocellulose degradation enzymes,, enzyme plate assays also indicated a significant increase in xylanase, a hemicellulosic degradation enzyme, in TEC-165. When grown on TEC medium containing 2-5% glucose and the color substrate AZCL xylan, glucose derepressed mutants, TEC-165, TEC-199, TEC-201, and TEC-207 colonies produced a more intense blue halo surrounding the strains compared to TEC-147 or TEC-193 (**Figure 10**), indicating more xylanase activity in the glucose derepressed mutants than the non-glucose derepressed parent strains. Similar results were observed with all other glucose derepressed mutants.

Thus glucose derepressed mutants combines both improved cellulase and hemicellulase enzyme production.

Conclusions of mutagenesis experiments

UV, NTG, NQO, and a combination of NTG+NQO were used to mutagenize strains from the 142 strain group (TEC-142 and TEC-147) and the 180 strain group (TEC-180, TEC-192, and TEC-193). The efficiency of mutagenesis was estimated by determining the increase in the frequency of mutants with an easily scorable phenotype (e.g. resistance to 5-FOA). Most effective UV and NTG treatments gave comparable mutation factors of -100. A combination of NTG and NQO gave higher killing and resulted in a much higher mutation factor of >100. Preselection of mutants for 2-deoxyglucose (DOG) resistance proved to be an effective method for obtaining glucose derepressed cellulase mutants. The best derepressed mutant from the 142 strain group was TEC-165 and was obtained from NTG+NQO treatment. TEC-165 produced in standard shake flask medium with addition of 5% glucose about 50-60% of the TEC activity that can be obtained in the same shake flask medium without addition of glucose; TEC-142 produces less than 1% in the presence of glucose. 10 L scale fermentations confirmed the glucose derepressed phenotype of strain TEC-165. So far the best glucose derepressed mutant of 180 strain group (15% better TEC production in batch fermentation than TEC-147) is TEC-200 and TEC-205. TEC production of either strains in shake flasks using glucose medium is 10-20% higher than TEC-165. 10 L scale fermentations confirmed the glucose derepressed phenotype of strain TEC-201, TEC-205, TEC-209, and TEC-210.

## EXAMPLE 14

This example describes the characterization of the cellulase-improved *T. emersonii* mutant TEC-147, TEC-165, TEC-180, and TEC-192 for increase levels of specific cellulase as determined by proteomics.

SDS PAGE analysis of supernatants of 10-liter Eschweiler batch fermentations of TEC-147, TEC-165, TEC-180, and TEC-192 coupled with identification and quantification by LC/MS/MS identification using an EST and whole genome sequence database (APEX) verified that the protein level of TEC cellulase enzymes had increased. Compared to the parent strain FBG-101 at least six specific cellulases were identified: cellobiohydrolase I (CBH I: molecular weight 48690 Daltons), cellobiohydrolase II (CBH II: molecular weight 46674 Daltons), three endoglucases (EG:, with molecular weight 24136 Daltons; EG/CEA, with molecular weight 36825 Daltons; and EG/CEB with molecular weight 51462 Daltons), and one beta-glucosidase (BG with molecular weight 94888 Daltons). Additionally identified were at least two hemicellulases, xylanase (molecular weight 43818 Daltons) and acetyl xylan esterase (AXE: molecular weight 32538 Daltons), and three putative protein that could assist in the degradation of lignocellulose, a EG/ GH61 protein (molecular weight 27007 Daltons), a swollenin-like protein (molecular weight 67911 Daltons), and a carbohydrate degrading protein (molecular weight 22993 Daltons) (**Table 9**).

Comparison of the TEC-101 extracellular protein profile with those profiles of the mutants shows important differences. All mutants produced more CBH I and CBH II than the control; the increases were generally in the range of 2-3 fold. Only TEC-147 and TEC192 had significantly higher EG/CEA protein levels. No difference was detected for EG/CEB, but a decrease in a third EG was observed (EG molecular weight 24136 Daltons). Variable levels of BG were observed compared to the TEC-101 control: a decrease in TEC-147, an increase in TEC-165, and no change in TEC-165 and TEC-192.

For hemicellulases, a significant increase in xylanase was observed in 3 of the 4 mutants compared to TEC-101, but not in TEC-192. An increase in acetyl xylan esterase was also detected, but was only significant for TEC-180. Finally for accessory proteins thought to assist cellulases in degradation of lignocellulose, three proteins showed significantly higher levels in the mutants than TEC-101: the EG/ GH61 protein, and the swollenin-like protein. The increases were 3-4-fold and >10-fold. Finally a carbohydrate degrading protein (molecular weight 22993 Daltons) was also shown to be significantly increased (1.8-fold or higher) in all mutants tested.

**Table 9.** Comparison of the relative cellulases and other protein levels (APEX) of *Talaromyces* strains TEC101, TEC-147, TEC-165, TEC-180, and TEC-192[a].

| Enzyme/ Protein | Mol wt (Daltons) | Strain | | | | |
|---|---|---|---|---|---|---|
| | | TEC-101 | TEC-147 | TEC-165 | TEC-180 | TEC-192 |
| CBH I | 48'690 | 4.3±0.1 | **8.0±0.4** | **8.2±0.2** | **8.6±1.4** | **9.7±0.4*** |
| CBH II | 46'674 | 2.6±0.7 | **7.3±1.2** | **7.0±0.7** | **6.2±0.5** | **6.9±0.8** |
| EG | 24'136 | 2.7±0.4 | **1.5±0.7** | **1.0±0.2** | **0.9±0.3** | **0.9±0.1** |
| EG/CEA | 36'825 | 1.2±0.2 | **3.0±0.9** | 3.1±2.5 | 3.0±1.6 | **3.6±1.1** |
| EG/CEB | 51'462 | 1.4±1.0 | 2.1±0.9 | 2.0±0.3 | 2.0±0.8 | 2.0±0.7 |
| Total EG (EG+EG/CEA+EG/CEB) | | 5.3 | 6.6 | 6.1 | 5.9 | 6.5 |
| BG | 94'888 | 1.2±0.1 | **0.9±0.1** | **1.6±0.3*** | **1.2±0.3*** | **1.2±0.2*** |
| Xylanase | 43'818 | 2.5±0.3 | **3.5±0.1** | **3.8±0.2*** | **3.5±0.5** | 3.3±1.5 |
| Acetyl xylan esterase | 32'538 | 7.9±5.9 | 11.1±3.7 | 10.3±1.1 | **14.7±2.1** | 9.6±7.3 |
| EG/GH61 | 27'007 | 2.8±0.3 | **8.6±1.5** | **9.4±5.4** | **8.1±3.7** | **10.8±2.3** |
| Swollenin-like protein | 67'911 | 0.1±0.0 | **1.4±0.4** | **1.4±0.5** | **1.2±0.1** | **1.5±0.4** |
| Carbohydrat e degrading protein | 22'993 | 1.6±0.9 | **3.4±1.5** | **3.6±1.3** | **3.1±1.3** | **3.2±0.2** |
| Total I[b] | | 18 ±1 | **36±1** | 37±3 | **34±1.5*** | **40±2.1*** |
| | | | | | | |
| Total II[c] | | 27±5 | **51±3** | 51±4 | **53±2.5** | **53±4** |
| | | | | | | |

(continued)

| Enzyme/ Protein | Mol wt (Daltons) | Strain | | | | |
|---|---|---|---|---|---|---|
| | | TEC-101 | TEC-147 | TEC-165 | TEC-180 | TEC-192 |
| EG/GH61/Total EG | | 0:53 | 1.30 | 1,54 | 1.37 | 1.66 |
| | | | | | | |
| Protease | 28'800 | 2.7±0.8 | 1.8±0.3 | 1.5±1.6 | **1.3±0.8** | **1.6±0.4** |
| Chitinase | 45'111 | 0.9±0.4 | **0.4±0.2** | **0.2±0.1** | **0.2±0.3** | **0.3±0.2** |
| | | | | | | |
| $FPU_{2\%}$/ml | | 5.1 | 17.3 | 18.5 | n.d. | 20.0 |

[a] Supernatants from 96 hr 10-liter Eschweiler batch fermentations were subjected to SDS-PAGE and their extracellular proteins were identified and quantified by LC/MS/MS identification using an EST and whole genome sequence database. All values are expressed in percentages of the proteins detected. Values in **bold** indicate that the levels in the descendent strains are statistically significantly different from the TEC-101 strain (p-value ≤ 0.05 in Student's T-test, 2-sided, unequal variance). Values with an asterisk indicate levels that change statistically significantly between TEC-147 and the descendent strains (p-value ≤ 0.05 in Student's T-test, 2-sided, unequal variance). TEC levels as $FPU_{2\%}$/ml from batch fermentations is shown at the bottom; n.d. not determined.
[b] Sum of cellulases and accessory proteins listed.
[c] Sum of cellulases, hemicellulases, and accessory proteins listed.

Practically all of these changes in protein levels occurred in the first HTS mutagenesis and screening that resulted in strain TEC-147. However, a further improvement in the abundance of xylanase was observed in TEC-165 (10%) and a further improvement of CBH I (20%) was observed in TEC-192. Also BG which was significantly decreased in TEC-147 relative to TEC-101, significantly increased in TEC165, TEC-180, and TEC-192 to levels found in TEC-101.

In total, the increase in protein content of these six cellulases, two hemicellulases, and three accessory proteins increased significantly from 27% in TEC-101 to over 50% in the mutants. However, just considering the six cellulase and three accessory proteins, a significant increase of TEC-192 compared to TEC-147 was found, again showing the second CSI campaign resulted in a further improvement in cellulase production.

**References:**

Brookes, P. 1990. The early history of the biological alkylating agents, 1918-1968. Mutat. Res. 233: 3-14.

Crueger, A. 1993. Mutagenesis, p. 5-45. In H.-J. Rehm, G. Reed, A. Pühler, and P. Stadler (ed.), Biotechnology, 2nd edition, Vol. 2 Genetic Fundamentals and Genetic Engineering. VCH, Weinheim, Germany.

Ginetti, F., M. Perego, A. M. Albertini, and A. Galizzi. 1996. Bacillus subtilis mutS mutL operon: identification, nucleotide sequence and mutagenesis. Microbiology 142:2021-2029.

Horiuchi, T., H. Maki, and M. Sekiguchi. 1989. Mutators and fidelity of DNA replication. Bull. Inst. Pasteur 87: 309-336.

Sasaki, M. and Y. Kurusu. 2004. Analysis of spontaneous base substitutions generated in mutator strains of Bacillus subtilis. FEMS Microbiol. Lett. 234:37-42.

Sasaki, M., Y. Yonemura, and Y. Kurusu. 2000. Genetic analysis of Bacillus subtilis mutator genes. J. Gen. Appl. Microbiol. 46:183-187.

Schaaper, R. M., and M. Radman. 1989. The extreme mutator effect of Escherichia coli mutD5 results from saturation of mismatch repair by excessive DNA replication errors. EMBO J. 8: 3511-3516.]

Vinci, V.A. and Byng, G. 1999. Strain improvement by non-recombinant methods, p. 103-113. In A.L. Demain and J.E. Davies (ed.), Manual of Industrial Microbiology and Biotechnology, 2nd edition, American Society for Microbiology Press, Washington, D.C., USA.

**Claims**

1. *Talaromyces* strain which has a cellulolytic activity of at least 6 FPU$_{2\%}$/ml, at least 7 FPU$_{2\%}$/ml, at least 8 FPU$_{2\%}$/ml, at least 10 FPU$_{2\%}$/ml at least 12 FPU$_{2\%}$/ml, at least 15 FPU$_{2\%}$/ml., at least 18 FPU$_{2\%}$/ml, or at least 20 FPU$_{2\%}$/ml.

2. *Talaromyces* strain according to claim 1, having an endoglucanase activity of at least 5000 WBCU/ml, at least 6000 WBCU/ml, at least 7000 WBCU/ml, at least 8000 WBCU/ml, at least 9000 WBCU/ml, or at least 10000 WBCU/ml.

3. *Talaromyces* strain according to claim 1 or 2, having an acid protease activity of at most 60 APU/ml, at most 50 APU/ml, at most 40 APU/ml, or at most 35 APU/ml.

4. *Talaromyces* strain according to claim any of claims 1 to 3, which has sporulation and produces a spore titer of at least 10$^6$ / ml.

5. *Talaromyces* strain according to claim any of claims 1 to 4, which is a *Talaromyces emersonii* strain.

6. *Talaromyces* strain according to any of claims 1 to 5, which is glucose de-repressed.

7. *Talaromyces* strain according to any of the claims 1 to 6, that produces xylanase when grown in the presence of glucose.

8. *Talaromyces* strain according to any of claims 1 to 7, which comprises a deletion or alteration of CreA.

9. *Talaromyces* strain according to claim any of claims 1 to 8, which is the strain with the deposition no. CBS 124902 or the strain with the deposition no. CBS127389 or a functionally equivalent mutant thereof.

10. Mutant *Talaromyces* strain produced by mutation of a wild-type *Talaromyces* strain, wherein the protein levels of one or more of the proteins CBH I, CBH II, EG/CEA, EG/CEB, Xylanase, Acetyl Xylan Esterase, Swollenin-like protein, EG/GH61 and/or carbohydrate degrading protein having a molecular weight of about 22993 Dalton is increased at least 1.5-fold, or at least 2-fold, relative to the protein level in the wild-type *Talaromyces* strain.

11. A method for the conversion of lignocellulosic material into useful product comprising the following steps: a) pretreatment of one or more lignocellulosic material to produce pretreated lignocellulosic material; b) enzymatic treatment of the pretreated lignocellulosic material to produce one or more sugar; c) converting the sugar into one or more useful product and d) separating the one or more useful product, wherein in step a), b) or c) a culture or the culture supernatant of a strain according to any of the claims 1 to 10 is used or added.

12. A method according to claim 11, wherein the lignocellulosic material is orchard primings, chaparral, mill waste, urban wood waste, municipal waste, logging waste, forest thinnings, short- rotation woody crops, industrial waste, wheat straw, oat straw, rice straw, barley straw, rye straw, flax straw, soy hulls, rice hulls, rice straw, corn gluten feed, sugar cane, corn stover, corn stalks, corn cobs, corn husks, miscanthus, sweet sorghum, canola stems, soybean stems, prairie grass, gamagrass, foxtail; sugar beet pulp, citrus fruit pulp, seed hulls, cellulosic animal wastes, lawn clippings, cotton, seaweed, softwood, poplar, pine, shrubs, grasses, wheat, sugar cane bagasse, corn, corn hobs, corn kernel, fiber from kernels, products and by-products from wet or dry milling of grains, municipal solid waste, waste paper, yard waste, herbaceous material, agricultural residues, forestry residues, waste paper, pulp, paper mill residues, branches, bushes, canes, an energy crop, forest, a fruit, a flower, a grain, a grass, a herbaceous crop, a leaf, bark, a needle, a log, a root, a sapling, a shrub, switch grass, a tree, a vegetable, fruit peel, a vine, sugar beet pulp, wheat midlings, oat hulls, hard or soft wood, organic waste material generated from an agricultural process, forestry wood waste, or a combination of any two or more thereof.

13. A method according to claim 11 or 12, wherein the useful product is one or more of ethanol, butanol, lactic acid, a plastic, an organic acid, a solvent, an animal feed supplement, a pharmaceutical, a vitamin, an amino acid, an enzyme or a chemical feedstock.

**14.** Process for the preparation of a *Talaromyces* mutant strain comprising the following steps:

a) Selecting and isolating one colony variant from a plurality of colony morphologies of a *Talaromyces* strain;
b) Subjecting the colony variant to mutagenesis, and screening of the resulting mutants for high cellulase activity;
c) Selecting and isolating from the mutants one or more high cellulase mutant strain; and, optionally:
d) Selecting and isolating one colony variant of a high cellulase mutant strain from a plurality of colony morphologies of high cellulase mutant strain;
e) Subjecting strains from the colony variant of step d) to mutagenesis, and screening of the resulting mutants for resistance to glucose analogs (e.g. 2-deoxy-D-glucose [DOG]);
f) Selecting and isolating from the mutants from step e) one or more high cellulase glucose de-repressed mutant strain;
g) Subjecting the improved cellulase activity mutant from step c) again with mutagenesis, and screening of the resulting mutants for higher cellulase activity; and optionally
h) Subjecting strains from step g) to mutagenesis, and screening of the resulting mutants for resistance to glucose analogs (e.g. 2-deoxy-D-glucose [DOG]);
i) Re-isolating a high cellulase glucose de-repressed mutant strain from the one or more high cellulase glucose de-repressed mutant strains from step h).

(A)

(B)          (C)          (D)

Fig. 1

Fig. 2

Fig. 3

Fig. 4

Fig. 5

Fig. 6

Fig. 7

TEC-147                    TEC-165

Fig. 8

**MUC    MUG**

Agar + glucose +cellulose

Agar + cellulose

**TEC-101**

**MUC    MUG**

Agar + glucose +cellulose

**Agar + cellulose**

**TEC-165**

Fig. 9

Fig. 10

# EUROPEAN SEARCH REPORT

**Application Number**

EP 15 15 3776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOLONEY A P ET AL: "Isolation of mutants of Talaromyces emersonii CBS 814.70 with enhanced cellulase activity", ENZYME AND MICROBIAL TECHNOLOGY, STONEHAM, MA, US, vol. 5, no. 4, 1 January 1983 (1983-01-01), pages 260-264, XP002398223, ISSN: 0141-0229 | 1-6,10, 14 | INV. C12N1/14 C12N9/42 C12R1/645 |
| Y | * page 260, column 2, paragraphs ISOLATION,OF,MUTANTS - page 261, column 1 * | 1-14 | |
| X | MORRISON J ET AL: "Cellulase production by Talaromyces emersonii CBS 814.70 and a mutant UV7 during growth on cellulose, lactose and glucose containing media", ENZYME AND MICROBIAL TECHNOLOGY, STONEHAM, MA, US, vol. 9, no. 7, 1 July 1987 (1987-07-01), pages 422-425, XP023689275, ISSN: 0141-0229 [retrieved on 1987-07-01] | 1-6 | |
| Y | * table 1 * | 1-14 | TECHNICAL FIELDS SEARCHED (IPC)<br>C12N<br>C12R |
| X | TUOHY M G ET AL: "PRODUCTION OF THERMOSTABLE XYLAN-DEGRADING ENZYMES BY TALAROMYCES-EMERSONII", BIORESOURCE TECHNOLOGY, ELSEVIER, GB, vol. 39, no. 2, 1 January 1992 (1992-01-01), pages 131-138, XP002330765, ISSN: 0960-8524 | 1-7 | |
| Y | * page 133, column 2; table 1 * | 1-14 | |

-/--

The present search report has been drawn up for all claims

| Place of search | Date of completion of the search | Examiner |
|---|---|---|
| Munich | 25 March 2015 | Mabit, Hélène |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................
& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 15 3776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TRACEY C MCCARTHY ET AL: "Comparison of wild-type and UV-mutant [beta]-glucanase-producing strains of Talaromyces emersonii with potential in brewing applications", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 32, no. 4, 1 April 2005 (2005-04-01), pages 125-134, XP019357720, ISSN: 1476-5535 | 1-5 | |
| Y | * page 128; figure 1; table 1 * | 1-14 | |
| X | ZHOU JIN ET AL: "Optimization of cellulase mixture for efficient hydrolysis of steam-exploded corn stover by statistically designed experiments", BIORESOURCE TECHNOLOGY, ELSEVIER BV, GB, vol. 100, no. 2, 1 January 2009 (2009-01-01), pages 819-825, XP002596453, ISSN: 0960-8524, DOI: 10.1016/J.BIORTECH.2008.06.068 [retrieved on 2008-09-03] * page 824 * | 11-13 | |
| A | BHAT M K: "Cellulases and related enzymes in biotechnology", BIOTECHNOLOGY ADVANCES, ELSEVIER PUBLISHING, BARKING, GB, vol. 18, no. 5, 1 August 2000 (2000-08-01), pages 355-383, XP004211815, ISSN: 0734-9750 * table 1 * | 1-14 | TECHNICAL FIELDS SEARCHED (IPC) |

-/--

The present search report has been drawn up for all claims

| Place of search | Date of completion of the search | Examiner |
|---|---|---|
| Munich | 25 March 2015 | Mabit, Hélène |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

## EUROPEAN SEARCH REPORT

Application Number

EP 15 15 3776

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MCHALE A ET AL: "The cellulolytic system of Talaromyces emersonii - Purification and characterization of the extracellular and intracellular beta-glucosidases", BBA ENZYMOLOGY, ELSEVIER, AMSTERDAM, NL, vol. 662, no. 1, 13 November 1981 (1981-11-13), pages 152-159, XP023514655, ISSN: 0005-2744 [retrieved on 1981-11-13] ----- | 1-14 | |

TECHNICAL FIELDS
SEARCHED (IPC)

The present search report has been drawn up for all claims

| Place of search | Date of completion of the search | Examiner |
|---|---|---|
| Munich | 25 March 2015 | Mabit, Hélène |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

           

& : member of the same patent family, corresponding document

EPO FORM 1503 03.82 (P04C01)

REFERENCES CITED IN THE DESCRIPTION

**Patent documents cited in the description**

- WO 9837179 A
- WO 2010018105 A

**Non-patent literature cited in the description**

- **KNOWLES et al.** *TIBTECH,* 1987, vol. 5, 255-261
- **SHULEIN.** *Methods Enzymol.,* 1988, vol. 160 (25), 234-243
- **NEVALAINEN ; PENTTILA.** *Mycota,* 1995, 303-319
- **SUURNAKKI et al.** *Cellulose,* 2000, vol. 7, 189-209
- **FREER.** *J. Biol. Chem.,* 1993, vol. 268 (13), 9337-9342
- **AUBERT ; WOOD et al.** *Methods in Enzymology,* 1988, vol. 160 (9), 87-116
- **COUGHLAN et al.** Comparative Biochemistry of Fungal and Bacterial Cellulolytic Enzyme Systems. *Biochemistry and Genetics of Cellulose Degradation,* 1988, 11-30
- **SALHEIMO et al.** *Eur. J. Biohem.,* 2002, vol. 269, 4202-4211
- **FOREMAN et al.** *J. Biol. Chem.,* 2003, vol. 278 (34), 31988-31997
- More Gene Manipulations in Fungi. Academic Press, 1991
- **LINDE, M. et al.** *Biomass and Bioenergy,* 2008, vol. 32, 326-332
- **SCHELL, D.J.** *Applied Biochemistry and Biotechnology,* 2003, vol. 105-108, 69-85
- Mutagenesis. **CERDÁ OLMEDO.** Phycomyces. CSH Laboratory Press, 1987, 341-344
- Mutation. **BOS, C.J. ; STADLER, D.** Fungal Genetics, Principles and Practice. Marcel Dekker, 1996, 28-31
- **BOS et al.** *Curr. Genet.,* 1992, vol. 21, 117-120
- **JERZY BAL et al.** *Mutation Research,* 1977, vol. 56, 153-156
- **PEBERDY.** *Arch Micro,* 1975, vol. 105, 201-205
- **PEBERDY.** *J. Gen. Microbiol.,* 1985, vol. 131, 2813-2823
- **BROOKES, P.** The early history of the biological alkylating agents. *Mutat. Res.,* 1990, vol. 233 (3-14), 1918-1968
- Mutagenesis. **CRUEGER, A.** Biotechnology. VCH, 1993, vol. 2, 5-45
- **GINETTI, F. ; M. PEREGO ; A. M. ALBERTINI ; A. GALIZZI.** Bacillus subtilis mutS mutL operon: identification, nucleotide sequence and mutagenesis. *Microbiology,* 1996, vol. 142, 2021-2029
- **HORIUCHI, T. ; H. MAKI ; M. SEKIGUCHI.** Mutators and fidelity of DNA replication. *Bull. Inst. Pasteur,* 1989, vol. 87, 309-336
- **SASAKI, M. ; Y. KURUSU.** Analysis of spontaneous base substitutions generated in mutator strains of Bacillus subtilis. *FEMS Microbiol. Lett.,* 2004, vol. 234, 37-42
- **SASAKI, M. ; Y. YONEMURA ; Y. KURUSU.** Genetic analysis of Bacillus subtilis mutator genes. *J. Gen. Appl. Microbiol.,* 2000, vol. 46, 183-187
- **SCHAAPER, R. M. ; M. RADMAN.** The extreme mutator effect of Escherichia coli mutD5 results from saturation of mismatch repair by excessive DNA replication errors. *EMBO J.,* 1989, vol. 8, 3511-3516
- Strain improvement by non-recombinant methods. **VINCI, V.A. ; BYNG, G.** Manual of Industrial Microbiology and Biotechnology. American Society for Microbiology Press, 1999, 103-113